(19) Europäisches Patentamt
European Patent Office
Office européen des brevets

(11) EP 3 217 932 B1

(12) **EUROPEAN PATENT SPECIFICATION**

(45) Date of publication and mention of the grant of the patent:
**08.03.2023 Bulletin 2023/10**

(21) Application number: **15859854.0**

(22) Date of filing: **10.11.2015**

(51) International Patent Classification (IPC):
***A61F 13/15*** (2006.01) ***A61F 13/44*** (2006.01)

(52) Cooperative Patent Classification (CPC):
**A61F 13/15; A61F 13/36; A61F 13/44**

(86) International application number:
**PCT/US2015/059806**

(87) International publication number:
**WO 2016/077259 (19.05.2016 Gazette 2016/20)**

(54) **SURGICAL ARTICLES AND METHODS FOR DETECTION**

CHIRURGISCHE ARTIKEL UND VERFAHREN ZUM NACHWEIS

ARTICLES CHIRURGICAUX ET PROCÉDÉS DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2014 US 201462078529 P**

(43) Date of publication of application:
**20.09.2017 Bulletin 2017/38**

(73) Proprietor: **Georgia State University Research Foundation, Inc.**
**Atlanta, GA 30303 (US)**

(72) Inventors:
- **HENARY, Maged**
  **Lawrenceville, Georgia 30043 (US)**
- **MALRAY, Jordan L.**
  **Greenville, SC 29615 (US)**

(74) Representative: **Lau, Sarah Jane et al**
**Kilburn & Strode LLP**
**Lacon London**
**84 Theobalds Road**
**London WC1X 8NL (GB)**

(56) References cited:
**WO-A1-2014/144624** **WO-A2-02/19918**
**US-A- 2 909 177** **US-A- 6 036 885**
**US-A1- 2008 255 403** **US-A1- 2011 163 854**
**US-A1- 2011 268 896** **US-A1- 2012 259 211**
**US-A1- 2014 248 213** **US-B1- 6 217 794**

- **D HARIHARAN ET AL: "Retained surgical sponges, needles and instruments", ANNALS OF THE ROYAL COLLEGE OF SURGEONS OF ENGLAND, vol. 95, no. 2, 1 March 2013 (2013-03-01), pages 87-92, XP055464965, GB ISSN: 0035-8843, DOI: 10.1308/003588413X13511609957218**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 217 932 B1

## Description

### CROSS-REFERENCE TO RELATED APPLICATIONS

This application claims the benefit of U.S. Provisional Application No. 62/078,529 filed November 12, 2014.

### FIELD

This disclosure relates generally to the detection of surgical articles.

### BACKGROUND

It has been estimated that the incidence of retained surgical items (RSIs) is about 1 in 1000 to about 1 in 1,500 abdominal operations and about 1 in 8000 to about 1 in 18,000 in-patient operations in the United States alone (Sowka M.P., Conn Med., 1981, 45:109-115 and Gawande A., et al., N Engl J Med., 2003, 348:229-235). In some cases, minor complications may occur relatively soon following a surgical procedure. Yet in others, serious complications such as fistula formation, sepsis, and even death can occur, with patients retaining the RSI inside of their body months or years after the procedure. The health, monetary, and morale-related repercussions of RSIs can be so severe that the ‘No Thing Left Behind® initiative was started in 2004 to help healthcare providers better understand the events that lead to RSI cases and to develop methods in an attempt to prevent them. Additionally, the legal ramifications are quite significant, with RSI cases falling under the doctrine of *res ipsa loquitur* ("the thing speaks for itself'), wherein the surgeon is automatically declared negligent once an RSI has been detected.

Strategic counting typically occur prior to and following surgical procedures to ensure the correct number of surgical articles are recovered. However, despite the attempts of the operating room staff, 80% of RSIs occur even after the count was called correct. Because of innate human error, three separate systems, x-ray, radio-frequency identification (RFID), and data-matrix-coded (DMC) sponges have been implemented in an attempt to limit retained surgical sponges.

X-ray tags affixed to surgical articles can be used solely or in conjunction with other methods to detect RSIs. However, even with this technology, false-negative results have been found in 10-30% of RSI cases. Even when this method is successful in finding lost surgical articles it is not an ideal approach as it exposes the patient to unnecessary and potentially harmful ionizing radiation. When RFID tags are used as a detection method, a chip or tag is placed on each article. If there is an incorrect count after surgery, a wand that detects radio-frequency waves is passed over the surgical site. However, there is a high cost in producing a large number of RFID chips/tags for this method. This is also the case for DMC sponges, as the hospital must buy the barcode printing, scanning and counting software/hardware required. Although these three methods aid in limiting the cases of RSIs, incidences still seem to slip through the cracks. There is therefore a need for improved methods of detecting RSIs.

WO2002/19918 discusses surgical articles, including needles, other hardware, and surgical supplies that include an element that fluoresces upon exposure to ultraviolet light. This quality makes the surgical articles more visible against their background, and facilitates the location of missing items that may be inadvertently dropped or retained during surgery, and also facilitates the inspection of items placed in surgery.

US2011/163854 discusses a method and apparatus for identifying and tracking surgical objects. More specifically, a method and apparatus for identifying and tracking surgical objects such as needles, scalpels, blades, sponges and instruments in a medical industry using an identifier encoded on a fluorescent paint attached to the surgical object combined with detectors and software capable of retrieving the identifying information on the identifier.

US2014/248213 discusses heptamethine cyanine dyes that possess both nuclear and near-infrared imaging capabilities. The dyes can be used for imaging, targeting and detecting tumors in patients.

WO2014/144624 discusses a medical material or surgical sponge including an integral construction having a plurality of micro-particle taggants that are UV or radio wave detectable. The sponge includes a fibrous, nonwoven fabric containing entangled fibers arranged in an interconnecting patterned relationship in a plane of the fabric, and at least one UV or radio wave detectable element positioned interiorly of the fibrous nonwoven fabric in the plane. The fibers of the nonwoven fabric may be intertwined about the micro-particle taggants.

### SUMMARY

The invention is set out in the appended claims.

Provided herein are surgical articles comprising a fluorophore selected from a near infrared (NIR) agent or a fluorescent protein, wherein the NIR agent has a maximum absorption at a wavelength of from 650 nm to 1,000 nm, wherein the fluorophore is affixed to the material covalently, hydrophobically, ionically, by hydrogen bond, or combinations thereof. Methods of detecting the surgical article inadvertently left within the body cavity of a subject are also provided.

In some aspects, the detection of surgical article inadvertently left within the body cavity of the subject can occur prior to closing the body cavity after a surgical procedure.

In some aspects, the surgical article can comprise a material selected from the group consisting of a fabric, a paper, a plastic material, a wood, a metal, a glass, a gel, a ceramic, a powder, or combinations thereof. Examples of surgical articles can include a laparotomy pad, a sponge, a gauze, a cottonoid, a surgical packing, a needle, a surgical tape, a surgical retractor, a clip, a suture thread, a staple, a knife blade, a safety pin, a scalpel, a clamp, a scissors, a hemostat, a tweezer, a forcep, a suction tip or tube, a scope, an ultrasound tissue disruptor, an asepto bulb, a central line guide wire, a catheter, and combinations thereof. In some cases, the surgical article can be an article capable of absorbing fluids within a body (e.g., a sponge or a gauze).

The fluorophore can be selected so as to possess photophysical properties that are compatible with detection of the article in the body cavity of a subject. The NIR agents have a maximum absorption at a wavelength of from 650 nm to 1,000 nm. In some aspects, the NIR agent is biocompatible. The biocompatible near infrared agent can be selected from the group consisting of a cyanine compound, a coumarin compound, a squarylium compound, a 4,4-difluoro-4-bora-3a,4a-diaza-s-indacene (BODIPY) compound, an oxazine compound, a rhodamine compound, a phthalocyanine, a porphyrin, a derivative thereof, or combinations thereof. In some aspects, the NIR agent can comprise at least one charged group under physiological conditions or is synthesized as a charged compound, wherein the charged group is selected from the group consisting of sulfonic acid, sulfuric acid, boric acid, carboxylic acid, phosphonic acids, phosphoric acid, thioacetic acid, thiols, thiosulphate, oxalic acids, nitro group, amino acids, amine, ammonium group, alkoxide, salts thereof, and mixtures thereof. In particular embodiments, the biocompatible NIR agent is indocyanine green. The fluorescent protein can have a maximum absorption at a wavelength of from 350 nm to 700 nm, such as from 450 nm to 500 nm.

The fluorophore is affixed to the surgical article covalently, hydrophobically, ionically, by hydrogen bond, or combinations thereof. In some aspects, the fluorophore can be incorporated into a core-shell nanoparticle. The core-shell nanoparticle may comprise a surface coating that is physically adsorbed or covalently bonded to the surgical article.

Methods of making the surgical articles are also provided. The method includes affixing the near infrared agent or fluorescent protein to a plurality of biocompatible fibers; and assembling the biocompatible fibers into the surgical article. The biocompatible fluorophore can be affixed using a group reactive with a functional group present in the biocompatible fiber. For example, the fluorophore can comprise an amino group, a hydroxyl group, an alkenyl group, an alkoxy group, a sulfonyl group, a carboxylic acid group, or combinations thereof. In some aspects, the method involves establishing a solvent system capable of swelling the biocompatible fibers, contacting the biocompatible fibers with the solvent system for a time adequate to swell the biocompatible fibers, and contacting the swollen biocompatible fibers with a solution of the near infrared agent or the fluorescent protein. Once the fluorophore is affixed to, adsorbed on, or absorbed within the fibers, the fibers can then be woven into a surgical article or formed into a non-woven surgical article.

Methods for detecting the surgical article within a subject by illuminating a first portion of the subject with an excitation light source, and observing a fluorescent signal in the subject are described herein. The fluorescent signal can be displayed on a computing device. The presence of an increased fluorescent signal, relative to a background staining intensity can indicate that the article is in the subject.

## BRIEF DESCRIPTION OF THE DRAWINGS

**Figures 1A-1D** are pictures of irradiated untreated lap pad (Figure 1A), lap pad treated with 1 nM indocyanine green compound (ICG; Figure 1B), lap pad treated with 1 μM ICG (Figure 1C), and treated with 1 mM indocyanine green compound (Figure 1D), at 690 nm with a NIR night vision monocular.

**Figures 2A-2B** are pictures of a irradiated untreated lap pad treated with pig's blood (Figure2) and an irradiated lap pad treated with $10^{-5}$ M ICG compound solution and pig's blood (Figure 2B). The lap pads were irradiated at 690 nm with a NIR night vision monocular.

**Figures 3A-3D** are pictures of an irradiated pig intestine (Figure 3A), an irradiated sponge treated with 1 μM ICG compound under the pig's intestine (Figure 3B), an irradiated pig intestine (Figure 3C) and an irradiated sponge treated with 1 mM indocyanine compound under/around the pig's intestine (Figure 3D). Irradiation was at 690 nm with a NIR night vision monocular.

**Figures 4A-4B** are pictures of an irradiated pig spleen (Figure 4A), an irradiated sponge treated with 1 mM indocyanine compound under the pig's spleen (Figure 4B). Irradiation was at 690 nm with a NIR night vision monocular.

**Figures 5A-5B** are pictures of a non-irradiated 0.64cm x 0.64 cm cottonoid (Figure 5A) and an irradiated 0.64cm x 0.64 cm cottonoid (Figure 5B) at 690 nm with a NIR night vision monocular in a faux-abdomen. The cottonoids were treated with 1 μM indocyanine green compound.

**Figures 6A-6B** are pictures of a non-irradiated 0.64 cm x 7.62 cm cottonoid (Figure 6A) and an irradiated 0.64 cm x 7.62 cm cottonoid (Figure 6B) at 690 nm with a NIR night vision monocular in a faux-abdomen. The cottonoids

were treated with 1 mM indocyanine green compound.

**Figures 7A-7C** are pictures of an irradiated 4 cm x 4 cm sponge (Figure 7A), sponge treated with 1 mM indocyanine green compound for 1 hour (Figure 7B), and treated with 1 mM indocyanine green compound for 24 hours (Figure 7C), at 780 nm with a NIR night vision monocular in a surgical site of a female canine after resection of the ovaries, oviducts, uterine horn, and uterus.

**Figure 8** is a graph showing the fluorescence intensity of the female canine's blood samples before sponge placement and various times after sponge placement.

**Figures 7A-7C** are pictures of an irradiated thermoplastic in a bath of porcine blood (Figure 9A) and a thermoplastic treated with 1 mM ICG in a bath of porcine blood (Figure 9B). Irradiation was at 780 nm with a NIR night vision monocular.

**Figures 10A-10D** are pictures of un-irradiated hemostats (Figure 10A), irradiated hemostat in a bath of porcine blood (Figure 10B), irradiated hemostat (dispersed along the grip) treated with 1 mM ICG in a bath of porcine blood (Figure 10C), and irradiated hemostat (focused at the grip) treated with 1 mM ICG in a bath of porcine blood (Figure 10D). Irradiation was at 780 nm with a NIR night vision monocular.

**Figures 11A-11F** are pictures of un-irradiated suture needles (Figure 11A), irradiated suture needle treated with 1 mM ICG (Figure 11B), un-irradiated suture needle treated with 1 mM ICG in porcine spleen, with needle point exposed (Figure 11C), irradiated suture needle treated with 1 mM ICG in a porcine spleen (Figure 11D), un-irradiated suture needle treated with 1 mM ICG in porcine spleen, with middle portion of needle exposed (Figure 11E), and irradiated suture needle treated with 1 mM ICG in porcine spleen (Figure 11F). Irradiation was at 780 nm with a NIR night vision monocular.

## DESCRIPTION

Provided herein are surgical articles that include a fluorophore such that the surgical article can absorb and/or emit light upon excitation). In some embodiments, the fluorophore can include a near infrared (NIR) agent such that the surgical article can absorb and/or emit light in the NIR region of the electromagnetic spectrum. NIR agents can absorb and/or fluoresce light in the NIR region of the electromagnetic spectrum. NIR agents also exhibit relatively low scattering, thus photons released from NIR agents when excited can penetration into and out of a tissue, for example up to 5 mm below the tissue surface. Given the relatively low absorbance and scatter of tissues in the NIR region, this characteristic allows a high signal-to background ratio for target identification. In addition, NIR light is a non-ionizing radiation and is safe at the fluence rates needed for *in vivo* imaging. In some embodiments, the fluorophore can include a fluorescent protein such that the surgical article can absorb and/or emit light in the visible and/or ultraviolet region of the electromagnetic spectrum.

The surgical articles comprise a fluorophore affixed to a surgical article. Methods of detecting a surgical article within the body cavity of a subject, such as those inadvertently left behind, are provided. In some aspects, the detection of surgical article within the body cavity of the subject can occur prior to closing the body cavity after a surgical procedure.

Before the present articles and methods are disclosed and described, it is to be understood that the aspects described below are not limited to specific near infrared agents, fluorescent proteins, or means of affixing the fluorophore to the surgical article, as such may, of course, vary. It is also to be understood that the terminology used herein is for the purpose of describing particular aspects only and is not intended to be limiting.

Also, throughout this specification, various publications are referenced.

### General Definitions

In this specification and in the claims that follow, reference will be made to a number of terms, which shall be defined to have the following meanings:

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a near infrared agent" includes mixtures of two or more such agents, reference to "the compound" includes mixtures of two or more such compounds, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be

understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed, then "less than or equal to" the value, "greater than or equal to the value," and possible ranges between values are also disclosed, as appropriately understood by the skilled artisan. For example, if the value "10" is disclosed, then "less than or equal to 10" as well as "greater than or equal to 10" is also disclosed. It is also understood that throughout the application data are provided in a number of different formats and that this data represent endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

It is understood that throughout this specification the identifiers "first" and "second" are used solely to aid in distinguishing the various components and steps of the disclosed subject matter. The identifiers "first" and "second" are not intended to imply any particular order, amount, preference, or importance to the components or steps modified by these terms.

**Chemical Definitions**

A weight percent (wt. %) of a component, unless specifically stated to the contrary, is based on the total weight of the formulation or composition in which the component is included.

The term "aliphatic" as used herein refers to a non-aromatic hydrocarbon group and includes branched and unbranched, alkyl, alkenyl, or alkynyl groups.

The term "alkyl" as used herein is a branched or unbranched saturated hydrocarbon group of 1 to 24 carbon atoms (e.g., 1 to 12 carbon atoms, 1 to 8 carbon atoms, 1 to 6 carbon atoms, or 1 to 4 carbon atoms), such as methyl, ethyl, n-propyl, isopropyl, n-butyl, isobutyl, t-butyl, pentyl, hexyl, heptyl, octyl, nonyl, decyl, dodecyl, tetradecyl, hexadecyl, eicosyl, tetracosyl, and the like. The alkyl group can also be substituted or unsubstituted. The alkyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halides, e.g., fluorine, chlorine, bromine, or iodine. The term "alkoxyalkyl" specifically refers to an alkyl group that is substituted with one or more alkoxy groups, as described below. When "alkyl" is used in one instance and a specific term such as "alkyl alcohol" is used in another, it is not meant to imply that the term "alkyl" does not also refer to specific terms such as "alkyl alcohol" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, *e.g.,* an "alkylcycloalkyl." Similarly, a substituted alkoxy can be specifically referred to as, *e.g.,* a "halogenated alkoxy," a particular substituted alkenyl can be, *e.g.,* an "alkenylalcohol," and the like. Again, the practice of using a general term, such as "cycloalkyl," and a specific term, such as "alkylcycloalkyl," is not meant to imply that the general term does not also include the specific term.

The term "alkoxy" as used herein is an alkyl group bound through a single, terminal ether linkage; that is, an "alkoxy" group can be defined as $-OA^1$ where $A^1$ is alkyl as defined above.

The term "alkenyl" as used herein is a hydrocarbon group of from 2 to 24 carbon atoms (e.g., 2 to 12 carbon atoms, 2 to 8 carbon atoms, 2 to 6 carbon atoms, or 2 to 4 carbon atoms) with a structural formula containing at least one carbon-carbon double bond. Asymmetric structures such as $(A^1A^2)C=C(A^3A^4)$ are intended to include both the E and Z isomers. This can be presumed in structural formulae herein wherein an asymmetric alkene is present, or it can be explicitly indicated by the bond symbol C=C. The alkenyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

The term "alkynyl" as used herein is a hydrocarbon group of 2 to 24 carbon atoms (e.g., 2 to 12 carbon atoms, 2 to 8 carbon atoms, 2 to 6 carbon atoms, or 2 to 4 carbon atoms) with a structural formula containing at least one carbon-carbon triple bond. The alkynyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol, as described below.

As used herein, the term "aryl," as well as derivative terms such as aryloxy, refers to groups that include a monovalent aromatic carbocyclic group of from 6 to 14 carbon atoms. Aryl groups can include a single ring or multiple condensed rings. In some embodiments, aryl groups include $C_6$-$C_{10}$ aryl groups. Examples of aryl groups include, but are not limited to, phenyl, biphenyl, naphthyl, tetrahydronaphtyl, phenylcyclopropyl, and indanyl. In some embodiments, the aryl group can be a phenyl, indanyl or naphthyl group. Aryl rings can be unsubstituted or substituted by one or more moieties as described below.

The term "heteroaryl", as well as derivative terms such as "heteroaryloxy", refers to a monovalent aromatic group of from 1 to 15 carbon atoms (*e.g.*, from 1 to 10 carbon atoms, from 2 to 8 carbon atoms, from 3 to 6 carbon atoms, or from 4 to 6 carbon atoms) having one or more heteroatoms within the ring. The heteroaryl group can include from 1 to 4 heteroatoms, from 1 to 3 heteroatoms, or from 1 to 2 heteroatoms. In some embodiments, the heteroaryl group can be a 5- or 6-membered aromatic ring. In some examples, the heteroatom(s) incorporated into the ring are oxygen, nitrogen, sulfur, or combinations thereof. When present, the nitrogen and sulfur heteroatoms can optionally be oxidized. Heteroaryl groups can have a single ring (*e.g.*, pyridyl or furyl) or multiple condensed rings provided that the point of attachment is through a heteroaryl ring atom. Example heteroaryl groups include pyridyl, piridazinyl, pyrimidinyl, pyrazinyl, triazinyl, pyrrolyl, indolyl, quinolinyl, isoquinolinyl, quinazolinyl, quinoxalinnyl, furanyl, thiophenyl, furyl, pyrrolyl, imidazolyl, oxazolyl, isoxazolyl, isothiazolyl, pyrazolyl benzofuranyl, and benzothiophenyl. Heteroaryl rings can be unsubstituted or substituted by one or more moieties as described below.

Aryl and heteroaryl groups may be unsubstituted or substituted with one or more chemical moieties. Examples of suitable substituents include, for example, hydroxy, nitro, cyano, formyl, alkyl, alkenyl, alkynyl, alkoxy, acyl, alkylthio, alkylsulfinyl, alkylsulfonyl, alkoxycarbonyl, carbamoyl, hydroxycarbonyl, alkylcarbonyl, aminocarbonyl, alkylaminocarbonyl, dialkylaminocarbonyl, provided that the substituents are sterically compatible and the rules of chemical bonding and strain energy are satisfied.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, etc. The term "heterocycloalkyl" is a cycloalkyl group as defined above where at least one of the carbon atoms of the ring is substituted with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol as described herein.

The term "cycloalkenyl" as used herein is a non-aromatic carbon-based ring composed of at least three carbon atoms and containing at least one double bound, *i.e.,* C=C. Examples of cycloalkenyl groups include, but are not limited to, cyclopropenyl, cyclobutenyl, cyclopentenyl, cyclopentadienyl, cyclohexenyl, cyclohexadienyl, and the like. The term "heterocycloalkenyl" is a type of cycloalkenyl group as defined above, and is included within the meaning of the term "cycloalkenyl," where at least one of the carbon atoms of the ring is substituted with a heteroatom such as, but not limited to, nitrogen, oxygen, sulfur, or phosphorus. The cycloalkenyl group and heterocycloalkenyl group can be substituted or unsubstituted. The cycloalkenyl group and heterocycloalkenyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, nitro, silyl, sulfo-oxo, sulfonyl, sulfone, sulfoxide, or thiol as described herein.

The term "alkylheteroaryl," as used herein, refers to a heteroaryl group that is bonded to a parent compound through a diradical alkylene bridge, $(-CH_2-)_n$, where n is 1-12 and where "heteroaryl" is as defined above.

The terms "heterocyclyl," "heterocyclic" and "heterocyclo" are used herein interchangeably, and refer to fully saturated or unsaturated, cyclic groups, for example, 3 to 7 membered monocyclic or 4 to 7 membered monocyclic; 7 to 11 membered bicyclic, or 10 to 15 membered tricyclic ring systems, having one or more heteroatoms within the ring. The heterocyclyl group can include from 1 to 4 heteroatoms, from 1 to 3 heteroatoms, or from 1 to 2 heteroatoms. In some examples, the heteroatom(s) incorporated into the ring are oxygen, nitrogen, sulfur, or combinations thereof. When present, the nitrogen and sulfur heteroatoms can optionally be oxidized, and the nitrogen heteroatoms can optionally be quaternized. The heterocyclyl group can be attached at any heteroatom or carbon atom of the ring or ring system and can be unsubstituted or substituted by one or more moieties as described for aryl groups above.

Exemplary monocyclic heterocyclic groups include, but are not limited to, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, 4-piperidonyl, pyridinyl, pyrazinyl, pyrimidinyl, pyridazinyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, triazolyl, triazinyl, and the like.

Exemplary bicyclic heterocyclic groups include, but are not limited to, indolyl, benzothiazolyl, benzoxazolyl, benzodioxolyl, benzothienyl, quinuclidinyl, quinolinyl, tetra-hydroisoquinolinyl, isoquinolinyl, benzimidazolyl, benzopyranyl, indolizinyl, benzofuryl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl]or furo[2,3-b]pyridinyl), dihydroisoindolyl, dihydroquinazolinyl (such as 3,4-dihydro-4-oxo-quinazolinyl), tetrahydroquinolinyl and the like.

Exemplary tricyclic heterocyclic groups include carbazolyl, benzidolyl, phenanthrolinyl, acridinyl, phenanthridinyl, xanthenyl, and the like.

The term "alkylheterocyclyl," as used herein, refers to a heterocyclyl group that is bonded to a parent compound through a diradical alkylene bridge, $(-CH_2-)_n$, where n is 1-12 and where "heterocyclyl" is as defined above. The term "heterocyclylalkyl," as used herein, refers to a heterocyclyl group, as defined above, which is substituted by an alkyl group, as defined above.

Heretrocyclyl and heteroaryl groups can be unsubstituted or substituted with one or more moieties chosen from alkyl, halo, haloalkyl, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamonyl, ester, phosphonyl, phosphinyl, phosphoryl, phosphine, thioester, thioether, acid halide, anhydride, oxime, hydrazine, carbamate, phosphoric acid, phosphate, phosphonate, or any other viable functional group that does not inhibit the biological activity of the compounds of the disclosure, either unprotected, or protected as necessary, as known to those skilled in the art, for example, as described in Greene, et al., Protective Groups in Organic Synthesis, John Wiley and Sons, Third Edition, 1999

The term "aldehyde" as used herein is represented by the formula -C(O)H. Throughout this specification "C(O)" is a short hand notation for C=O.

The terms "amine" or "amino" as used herein are represented by the formula $NA^1A^2A^3$, where $A^1$, $A^2$, and $A^3$ can be, independently, hydrogen, an alkyl, alkenyl, alkynyl, aryl, heteroaryl, cycloalkyl, cycloalkenyl, heterocycloalkyl, or heterocycloalkenyl group described above.

The term "carboxylic acid" as used herein is represented by the formula -C(O)OH. A "carboxylate" as used herein is represented by the formula $-C(O)O^-$.

The term "ester" as used herein is represented by the formula $-OC(O)A^1$ or $-C(O)OA^1$, where $A^1$ can be an alkyl, alkenyl, alkynyl, aryl, heteroaryl, cycloalkyl, cycloalkenyl, heterocycloalkyl, or heterocycloalkenyl group described above.

The term "ether" as used herein is represented by the formula $A^1OA^2$, where $A^1$ and $A^2$ can be, independently, an alkyl, alkenyl, alkynyl, aryl, heteroaryl, cycloalkyl, cycloalkenyl, heterocycloalkyl, or heterocycloalkenyl group described above.

The term "ketone" as used herein is represented by the formula $A^1C(O)A^2$, where $A^1$ and $A^2$ can be, independently, an alkyl, alkenyl, alkynyl, aryl, heteroaryl, cycloalkyl, cycloalkenyl, heterocycloalkyl, or heterocycloalkenyl group described above.

The term "halogen" as used herein refers to the halide fluorine, chlorine, bromine, and iodine.

The term "hydroxyl" as used herein is represented by the formula -OH.

The term "nitro" as used herein is represented by the formula $-NO_2$.

The term "alkylthio," as used herein, refers to alkyl-S-, wherein alkyl refers to an alkyl group, as defined above. Similarly, the term "cycloalkylthio," refers to cycloalkyl-S- where cycloalkyl are as defined above.

The term "alkylsulfinyl," as used herein, refers to alkyl-S(O)-, wherein alkyl refers to an alkyl group, as defined above.

The term "alkylsulfonyl," as used herein, refers to alkyl-$S(O)_2$-, wherein alkyl is as defined above.

The terms "alkylamino" and "dialkylamino," as used herein, refer to alkyl-NH- and $(alkyl)_2N$- groups, where alkyl is as defined above.

The terms "alkylcarbonyl," "alkoxycarbonyl," "alkylaminocarbonyl," and "dialkylaminocarbonyl," as used herein, refer to alkyl-C(O)-, alkoxy-C(O)-, alkylamino-C(O)- and dialkylamino-C(O)- respectively, where alkyl, alkoxy, alkylamino, and dialkylamino are as defined above.

"R," "R'," "R"," "R‴," and "A" as used herein can, independently, possess one or more of the groups listed above. For example, if R is a straight chain alkyl group, one of the hydrogen atoms of the alkyl group can optionally be substituted with a hydroxyl group, an alkoxy group, an amine group, an alkyl group, a halide, and the like. Depending upon the groups that are selected, a first group can be incorporated within second group or, alternatively, the first group can be pendant (*i.e.*, attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen, can have hydrogen substituents and/or any permissible substituents of organic compounds described herein which satisfy the valences of

the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, *e.g.*, a compound that does not spontaneously undergo transformation such as by rearrangement, cyclization, elimination, etc.

The chemical groups described herein can be unsubstituted or substituted with one or more moieties chosen from alkyl, halo, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamonyl, ester, phosphonyl, phosphinyl, phosphoryl, phosphine, thioester, thioether, acid halide, anhydride, oxime, hydrazine, carbamate, phosphoric acid, phosphate, phosphonate, or any other viable functional group that does not inhibit the biological activity of the compounds of the disclosure, either unprotected, or protected as necessary, as known to those skilled in the art, for example, as described in Greene, et al., Protective Groups in Organic Synthesis, John Wiley and Sons, Third Edition, 1999.

The term "fluorophore" as used herein refers to a functional group and/or a molecule containing the functional group which will absorb energy of a specific wavelength and re-emit energy at a different wavelength.

It is understood that throughout this specification the identifiers "first" and "second" are used solely to aid in distinguishing the various components and steps of the disclosed subject matter. The identifiers "first" and "second" and the like are not intended to imply any particular order, amount, preference, or importance to the components or steps modified by these terms.

Reference will now be made in detail to specific aspects of the disclosed materials, compounds, compositions, articles, and methods, examples of which are illustrated in the accompanying Examples.

**Surgical Articles**

The surgical articles described herein include any surgical sponge or absorbent material, hardware, instrument, tool, or device that is used during a surgical procedure. Such articles can comprise any material selected from the group consisting of fabric, paper, plastic material, wood, metal, glass, and combinations thereof. In some embodiments, the surgical article can include an item capable of absorbing fluids within a body. For example, the surgical article can comprise a woven or non-woven fabric. The fabric can be formed from a natural fiber, a synthetic fiber, or combinations thereof. In some embodiments, the fabric can comprise fibers selected from the group consisting of cellulose, protein, nylon, triacetate, polyester, and combinations thereof. The fabric can comprise entangled fibers arranged in an interconnecting relationship in the fabric.

Examples of surgical articles can include absorbent items, sharps, vascular items, rubber bands, surgical catheters, additional and parts of instruments (including loan instruments), disposable instruments, and disposable retraction devices. Specific examples of surgical articles can include but are not limited to laparotomy pad, a sponge, a swab (such as a Raytec® swab), a gauze, surgical patties and strips, a peanut sponge, a stroll, a cotton wool ball, a prep ball, a surgical packing, a needle, a hypodermic needle including cap, a plegia needle, a surgical tape, a surgical retractor, a clip, a suture thread, a staple, a detachable blade, a diathermy tip, a safety pin, a scalpel, a clamp, a scissors, a hemostat, a tweezer, a forcep, a suction tip or tube, a scope, an ultrasound tissue disruptor, an asepto bulb, a central line guide wire, a catheter, a vessel loop, a snugger, a cardiac snare, tape, a ligareel, a ligaboot, clip cartridge, disposable bulldog clamp, a feeding tube that is not intended to be left as drain, a sucker, a fishhook, a visceral retractor, and combinations thereof.

**Near Infrared (NIR) Agents**

The NIR agents described herein have an absorption spectrum maximum in the NIR region of the electromagnetic spectrum. The absorption maximum of the NIR agent is from about 650 nm to about 1,000 nm, for example from about 700 nm to about 1,000 nm, from about 750 nm to about 1,000 nm, or from about 800 nm to about 1,000 nm.

The NIR agents described herein can have an emission spectrum maximum in the NIR region of the electromagnetic spectrum. The emission maximum of the NIR agent can be 700 nm or greater, 750 nm or greater, or 800 nm or greater. In some examples, the emission maximum of the NIR agent can be from about 650 nm to about 1,000 nm, from about 700 nm to about 1,000 nm, from about 750 nm to about 1,000 nm, or from about 800 nm to about 1,000 nm.

The NIR agent can be any suitable chromophore that can emit energy in the NIR region of the electromagnetic spectrum when excited with electromagnetic radiation. In some aspects, the NIR agent can be a NIR dye. In some embodiments, the near infrared agent can be selected from the group consisting of a cyanine compound, a coumarin compound, a squarylium compound, a 4,4-difluoro-4-bora-3a,4a-diaza-s-indacene (BODIPYs) compound, an oxazine compound, a rhodamine compound, a phthalocyanine, a porphyrin derivative, a derivative thereof, or combinations thereof.

The NIR agent can be a cyanine compound having a structure defined by Formula I, or a salt, a hydrate, or a

derivative thereof

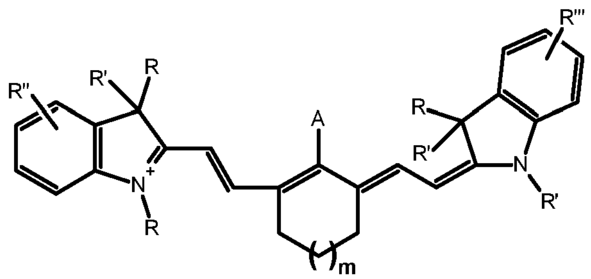

Formula I,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and A, R" and R‴ are independently selected from the group consisting of hydrogen; halogen, alkyl, $-OR^1$; $-NR^1R^2$; $-NR^1R^2R^3$; $-NO_2$; $-CF_3$; -CN; $-C_2R'$; -SR'; $-N_3$; C(=O) $R^1$; $-C(=O)OR^1$; - $OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; - $O(CR^1R^2)_rNR^2SO_2R^1$; $-OC(=O)NR^1R^2$; $-NR^1C(=O)OR^2$; $-SO_2R^1$; $-SO_2NR^1R^2$; and $-NR^1SO_2R^2$; wherein $R^1$, $R^2$, and $R^3$ are individually hydrogen; substituted or unsubstituted alkyl; alkenyl; alkynyl; cycloalkyl; heterocyclyl; cycloalkenyl, heterocycloalkenyl, aryl, heteroaryl, arylalkyl, heteroalkyls, aryl, alkylaryl, alkylheteroaryl; and wherein m is 0, 1, 2, or 3, and r is an integer from 1 to 6.

In some embodiments, A is selected from the group consisting of substituted or unsubstituted cycloalkyl; heterocyclyl; cycloalkenyl, heterocycloalkenyl, aryl, heteroaryl, arylalkyl, heteroalkyls, aryl, alkylaryl, and alkylheteroaryl.

The NIR cyanine compound can be an indocyanine having a structure defined by Formula II, or a salt, a hydrate thereof, or a derivative thereof:

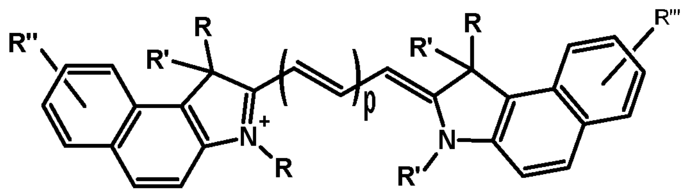

Formula II,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and R" and R‴ are independently selected from the group consisting of hydrogen; halogen, alkyl, $-OR^1$; $-NR^1R^2$; $-NR^1R^2R^3$; $-NO_2$; $-CF_3$; -CN; $-C_2R'$; -SR'; $-N_3$; C(=O) $R^1$; $-C(=O)OR^1$; - $OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; - $O(CR^1R^2)_rNR^2SO_2R^1$; $-OC(=O)NR^1R^2$; $-NR^1C(=O)OR^2$; $-SO_2R^1$; $-SO_2NR^1R^2$; and $-NR^1SO_2R^2$; wherein $R^1$, $R^2$, and $R^3$ are individually hydrogen; substituted or unsubstituted alkyl; alkenyl; alkynyl; cycloalkyl; heterocyclyl; cycloalkenyl, heterocycloalkenyl, aryl, heteroaryl, arylalkyl, heteroalkyls, aryl, alkylaryl, alkylheteroaryl; and wherein p is an integer from 0 to 3, and r is an integer from 1 to 6.

The indocyanine compound can have a structure defined by Formula III, or a salt, a hydrate, or a derivative thereof,

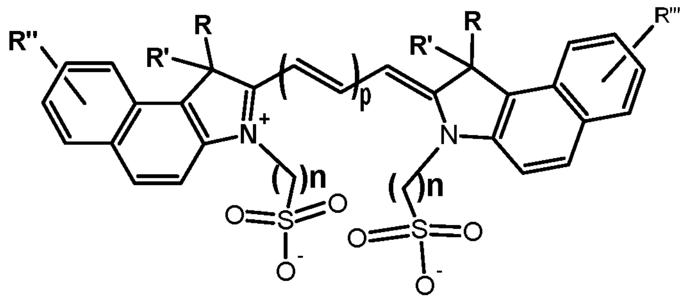

Formula III,

wherein R, R', R", and R‴ are as defined above, and wherein p is an integer from 0 to 3, n is an integer from 1 to 6, and r is an integer from 1 to 6.

The NIR agent can be a squarylium compound having a structure defined by Formula IV, or a salt, a hydrate, or a derivative thereof,

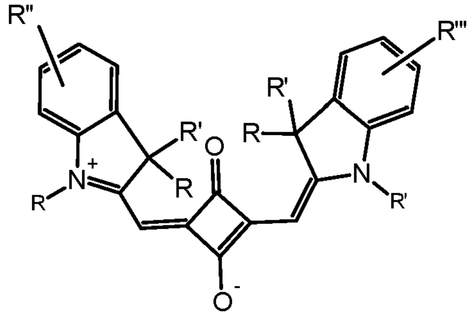

Formula IV,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and R" and R‴ are independently selected from the group consisting of hydrogen; halogen; alkyl, $-OR^1$; $-NR^1R^2$; ;$-NO_2$; $-CF_3$; -CN; $-C_2R^1$; $-SR^1$; $-N_3$; $C(=O)R^1$; $-C(=O)OR^1$; $-OC(=O)R^1$; - $O(CR^1R^2)_rC(=O)R^1$; $-C(=O)NR^1R^2$; $-NR^1C(=O)R^2$; $-O(CR^1R^2)_rNR^2C(=O)R^1$; - $O(CR^1R^2)_rNR^2SO_2R^1$; $-OC(=O)NR^1R^2$; $-NR^1C(=O)OR^2$; $-SO_2R^1$; $-SO_2NR^1R^2$; and $-NR^1SO_2R^2$; wherein $R^1$ and $R^2$ are individually hydrogen; substituted or unsubstituted alkyl; substituted or unsubstituted alkenyl; substituted or unsubstituted alkynyl; substituted or unsubstituted cycloalkyl; substituted or unsubstituted heterocyclyl; substituted or unsubstituted cycloalkenyl, substituted or unsubstituted heterocycloalkenyl, substituted or unsubstituted aryl, substituted or unsubstituted heteroaryl, substituted or unsubstituted arylalkyl, substituted or unsubstituted heteroalkyl, or substituted or unsubstituted alkylheteroaryl; and r is an integer from 1 to 6.

In some embodiments, the R and/or R' on the nitrogen atom of the squarylium compound can include a positively charged group, a group that can be converted to a positively charged group under physiological conditions, and/or a hydrogen atom capable of hydrogen bonding. Without wishing to be bound by theory, interaction between the positively charged group, the group that can be converted to a positively charged group under physiological conditions, or the hydrogen atom capable of hydrogen bonding can interact with the oxyanion group of the cyclobutene ring to stabilize the squarylium compound. In particular, squarylium compounds by definition have a low HOMO-LUMO band gap, and thus they may be susceptible to chemical attack. The squarylium compounds may be susceptible to nucleophilic attack on the electron-deficient cyclobutene ring. Accordingly, a positively charged group can interact electrostatically with the oxyanion, thereby forming a stable structure. Similarly, a hydrogen atom capable of forming a hydrogen bond can also interact favorably with the oxyanion group. As a result, the chemical stability as well as the photophysical properties of the squarylium compound can be improved.

In certain embodiments, the squarylium compound can have a structure defined by Formula IVa, or a salt, a

hydrate, or a derivative thereof,

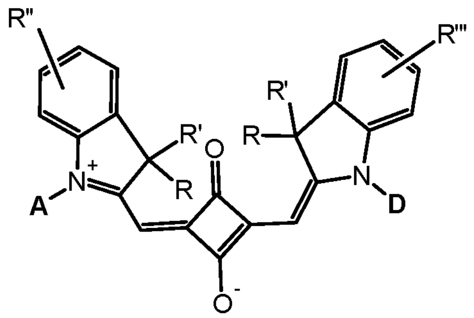

Formula IVa,

wherein A comprises a positively charged group, a group that can be converted to a positively charged group under physiological conditions, or a hydrogen atom capable of hydrogen bonding; D comprises a functional group reactive with the material used to make the surgical article; and R, R', R", and R'" are as described herein.

In certain embodiments, A can include a metal, ammonium, imine, alkyl amine, alkylene amine, or alkanol amine. In certain embodiments, D can include an amino group, a hydroxyl group, an alkenyl group, an alkoxy group, a sulfonyl group, phosphonate, a carboxylic acid group, or combinations thereof.

In some examples, the squarylium compound can have a structure defined by Formula IVb, or a salt, a hydrate, or a derivative thereof,

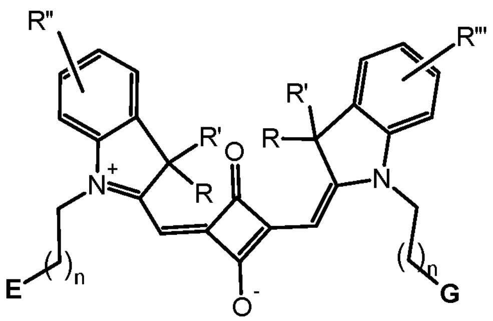

Formula IVb,

wherein E can include $-NR^1R^2R^3$, $-C(R^1)=NR^2$, a divalent or trivalent metal, a transition metal, wherein $R^1$, $R^2$, and $R^3$ are independently present or absent and are independently selected from hydrogen or substituted or unsubstituted $C_1$-$C_6$ alkyl group, $C_2$-$C_6$ alkenyl group, cycloalkyl, aryl, or aralkyl;
G can include an amino group, a hydroxyl group, an alkenyl group, an alkoxy group, a sulfonyl group, phosphonate, a carboxylic acid group, or combinations thereof; and
wherein n is independently 1, 2, 3, 4, 5, or 6.

In some embodiments, E can be selected from the group consisting of $-NH_2$, ammonium, methylamine, dimethylamine, trimethylamine, ethylamine, diethylamine, trimethylamine, propylamine, dipropylamine, tripropylamine, ethanolamine, methylethanolamine, methyldiethanolamine, dimethylethanolamine, propanolamine, ethylenediamine, diethylenetriamine, piperazine, aminoethylpiperazine, propylenediamine, and the like. In some embodiments, E can include a metal selected from magnesium, calcium, strontium, barium, chromium, manganese, iron, copper, cobalt, zinc, aluminum, or an aluminum cation of the formula $(R_1R_2Al)^{+1}$ (wherein each of $R_1$ and $R_2$ are as described herein).

The NIR agent can be a BODIPY compound having a structure defined by Formula V, or a salt, a hydrate, or a derivative thereof,

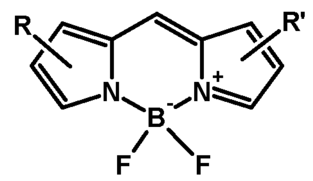

Formula V,

wherein R and R' are independently selected from the group consisting of hydrogen; halogen; $-OR^1$; $-NR^1R^2$; ;$-NO_2$; $-CF_3$; -CN; $-C_2R^1$; $-SR^1$; $-N_3$; $C(=O)R^1$; $-C(=O)OR^1$; - $OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; - $O(CR^1R^2)_rNR^2SO_2R^1$; $-OC(=O)NR^1R^2$; $-NR^1C(=O)OR^2$; $-SO_2R^1$; $-SO_2NR^1R^2$; and $-NR^1SO_2R^2$; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and

wherein $R^1$ and $R^2$ are individually hydrogen; substituted or unsubstituted alkyl; substituted or unsubstituted alkenyl; substituted or unsubstituted alkynyl; substituted or unsubstituted cycloalkyl; substituted or unsubstituted heterocyclyl; substituted or unsubstituted cycloalkenyl, substituted or unsubstituted heterocycloalkenyl, substituted or unsubstituted aryl, substituted or unsubstituted heteroaryl, substituted or unsubstituted arylalkyl, substituted or unsubstituted heteroalkyl, or substituted or unsubstituted alkylheteroaryl; and r is an integer from 1 to 6.

The NIR agent can be an oxazine compound having a structure defined by Formula VI, or a salt, a hydrate, or a derivative thereof,

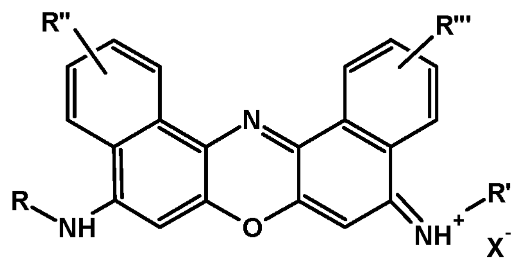

Formula VI,

wherein R, R', R", and R‴ are independently selected from the group consisting of hydrogen; halogen; $-OR^1$; $-NR^1R^2$; ;$-NO_2$; $-CF_3$; -CN; $-C_2R^1$; $-SR^1$; $-N_3$; $C(=O)R^1$; $-C(=O)OR^1$; - $OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; - $O(CR^1R^2)_rNR^2SO_2R^1$; $-OC(=O)NR^1R^2$; $-NR^1C(=O)OR^2$; $-SO_2R^1$; $-SO_2NR^1R^2$; and $-NR^1SO_2R^2$; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and

wherein $R^1$ and $R^2$ are individually hydrogen; substituted or unsubstituted alkyl; substituted or unsubstituted alkenyl; substituted or unsubstituted alkynyl; substituted or unsubstituted cycloalkyl; substituted or unsubstituted heterocyclyl; substituted or unsubstituted cycloalkenyl, substituted or unsubstituted heterocycloalkenyl, substituted or unsubstituted aryl, substituted or unsubstituted heteroaryl, substituted or unsubstituted arylalkyl, substituted or unsubstituted heteroalkyl, or substituted or unsubstituted alkylheteroaryl; X is a negatively charged counter ion including, but is not limited to halogens, tosylates, perchlorates, and r is an integer from 1 to 6.

In some examples, the NIR agent can comprise a moiety comprising a charged group or can be converted to a charged group under physiological conditions. The charged group can be a cation or an anion. Particular examples of cationic moieties that can be included in the NIR agents are moieties that contain nitrogen or phosphorus atoms. Nitrogen atom-containing groups can exist as a neutral compound or can be converted to positively-charged quaternary ammonium species, for example, through alkylation or protonation of the nitrogen atom. Similarly, the phosphorous

atom-containing groups can exist as a neutral compound or can be converted to positively-charged quaternary phosphonium species, for example, through alkylation or protonation of the phosphorous atom. Particular examples of anionic moieties that can be included in the NIR agents are sulfonic acid, sulfuric acid, boric acid, carboxylic acid, phosphonic acids, phosphoric acid, thioacetic acid, thiols, thiosulphate, oxalic acids, nitro group, amino acids, alkoxide, salts thereof, and mixtures thereof. In some examples, the NIR agent can be zwitterionic. In some examples, R, R', R", and R‴ can independently comprise a charged group or a group that can be converted to a charged group under physiological conditions. For example, R, R', R", and R‴ can independently be an alkyl group terminated with a charged group or a group that can be converted to a charged group under physiological conditions, such as - $(CH_2)_nNR^1R^2$, wherein n can be 1, 2, 3, 4, 5, or 6 and $R^1$ and $R^2$ are as described above.

The NIR agent can comprises a reactive group. The reactive group can be a functional group reactive with the material used to make a surgical article. For example, the reactive group can be a functional group reactive with a polymer used to make a surgical article. In some examples, the reactive group includes an amino group, a hydroxyl group, an alkenyl group, an alkoxy group, a sulfonyl group, a carboxylic acid group, or combinations thereof.

**Fluorescent Proteins**

The fluorescent proteins described herein can have an absorption spectrum maximum in the visible and/or ultraviolet region of the electromagnetic spectrum. The absorption maximum of the fluorescent proteins can be 450 nm or greater, such as 460 nm or greater, 480 nm or greater, or 490 nm or greater. In some examples, the absorption maximum of the fluorescent proteins can be from about 450 nm to about 500 nm. In some embodiments, the fluorescent proteins can have an absorption maximum in the blue region of the electromagnetic spectrum. The fluorescent proteins described herein can have an emission spectrum maximum in the visible region of the electromagnetic spectrum. The emission maximum of the fluorescent proteins can be 350 nm or greater, 400 nm or greater, 450 nm or greater, 500 nm or greater, 550 nm or greater, 600 nm or greater, or 650 nm or greater. In some examples, the emission maximum of the fluorophore can be from about 350 nm to about 700 nm, from about 400 nm to about 700 nm, or from about 400 nm to about 600 nm. In some embodiments, the fluorescent proteins can be any suitable chromophore that can emit energy in the visible region of the electromagnetic spectrum when excited with electromagnetic radiation.

A broad range of fluorescent protein genetic variants have been developed over the past several years that feature fluorescence emission spectral profiles spanning almost the entire visible light spectrum. Such variants of the GFP gene have been found useful to enhance expression and to modify excitation and fluorescence. Extensive mutagenesis efforts in the original jellyfish protein have resulted in fluorescent probes that range in color from blue to yellow. For example, substitution of a serine at position 65 to either alanine, glycine, isoleucine, or threonine results in mutant GFPs with a shift in excitation maxima and greater fluorescence than wild type protein when excited at 488 nm (see, e.g, Heim et al, 1995, Nature 373:663-664; U.S. Pat. No. 5,625,048; Delagrave et al, 1995, Biotechnology 13:151-154; Cormacketal, 1996, Gene 173:33-38; and Cramer et al, 1996, Nature Biotechnol. 14:315-319). Longer wavelength fluorescent proteins, emitting in the orange and red spectral regions, have been developed from the marine anemone *Discosoma striata* and reef corals belonging to the class Anthozoa. Still other species produce similar proteins having cyan, green, yellow, orange, red, and far-red fluorescence emission. A fluorescent protein, as used herein, includes wild type green fluorescent protein and its variants, as well as fluorescent proteins and variants from other species. Such fluorophores are many, and are known to those of skill in the art.

In some aspects, the fluorescent proteins can be selected from the group consisting of a green fluorescent proteins (GFP) such as EGFP, emerald, superfolder GFP, azami green, mWasabi, tagGFP, turboGFP, acGFP, zsGreen, t-sapphire; blue fluorescent proteins (BFP) such as EBFP, EBFP2, azurite, GFP2, GFP10, and mTagBFP; cyan fluorescent p (CFP) such as ECFP, mECFP, cerulean, cyPet, am Cyan 1, midori-Ishi cyan, tagCFP, mCFPmm, and mTFP1 (Teal); yellow fluorescent proteins (CFP) such as EYFP, topaz, venus, mCitrine, YPet, tagYFP, phiYFP, zsYellow1, and mBanana; orange fluorescent proteins (OFP) such as kusabira orange, kusabira orange2, mOrange, mOrange2, dTomato, dTomato-tandem, tagRFP, tagRFP-T, dsRed, dsRed2, dsRed-express (T1), dsRed-monomer, and mTangerine; and red fluorescent proteins (RFP) such as mRuby, mApple, mStrawberry, AsRed2, mRFP1, JRed, mCherry, HcRed1, mRaspberry, dKeima-tandem, HcRed-tandem, mPlum, tdTomato, and AQ143; saphhire-type proteins such as sapphire, T-sapphire, and mAmetrine; near infrared proteins such as iFP1.4, iRFP713, iRFP670, iRFP682, iRFP702, and iRFP720.

The fluorophore can exhibit a large stokes shift. Stoke shift refers to the difference (in wavelength or frequency units) between absorbance spectrum maximum and the emission spectrum maximum of the same electronic transition. Typically, the wavelength of maximum fluorescence emission is longer than that of the exciting radiation, i.e., the wavelength of maximum absorbance. A large stoke shift can be advantageous since the light produced by emission can be more easily distinguished from the light used for excitation. In some cases, fluorophores can have a stoke shift of 10 nm or greater, such as 20 nm or greater, 30 nm or greater, or 40 nm or greater. In some examples, the stoke shift of the fluorophore can be from about 10 nm to about 200 nm or from about 20 nm to about 100 nm.

The fluorophore can exhibit a high quantum yield. Quantum yield refers to the ratio of emitted electrons to

incident photons; that is, the fraction (or percentage) of incoming photons which result in a photoemitted electron. The quantum yield of the fluorophore can be low in an aqueous environment. In some embodiments, the quantum yield of the fluorophore in water can be about 10% or greater, such as about 15% or greater, or about 20% or greater. In some examples, the quantum yield of the fluorophore can be from about 10% to about 50% or from about 10% to about 40%.

The fluorophore can exhibit a high extinction coefficient. Extinction coefficient as used herein refers to the molar extinction coefficient (also referred to as the "molar absorption coefficient" or "molar absorptivity") and is a measurement of how strongly a chemical species absorbs light at a given wavelength on a molar basis. In some embodiments, the fluorophore can absorb a relatively high amount of the radiation to which the agent is exposed. The molar extinction coefficient of the fluorophore can be about 15,000 $M^{-1}cm^{-1}$ or greater, such as about 20,000 $M^{-1}cm^{-1}$ or greater, or about 30,000 $M^{-1}cm^{-1}$ or greater. In some examples, the molar extinction coefficient of the fluorophore can be from about 15,000 $M^{-1}cm^{-1}$ to about 300,000 $M^{-1}cm^{-1}$ or from about 15,000 $M^{-1}cm^{-1}$ to about 150,000 $M^{-1}cm^{-1}$. The product of the quantum yield and the molar extinction coefficient can be used as a guide in determining an agent's efficiency in converting incident radiation to fluorescence.

The fluorophore can be biocompatible. "Biocompatible" or "biologically compatible", as used herein, generally refers to fluorophores that are, along with any metabolites or degradation products thereof, generally non-toxic to cells and tissues, and which do not cause any significant adverse effects to cells and tissues when cells and tissues are incubated (*e.g.*, cultured) in their presence. In some embodiments, cells and/or tissues such as the liver, muscles, heart, blood, lung, gastrointestinal tract, and/or spleen exhibit low uptake of the fluorophore post administration. For example, in some aspects, the fluorophores exhibit renal clearance, i.e., the agent is taken up via kidney filtration. In one embodiment, the fluorophores exhibit rapid clearance from the blood. For example, in some embodiments, the kidney exhibit high compound uptake at early time points of about 10 hours or less, about 9 hours or less, about 8 hours or less, about 7 hours or less, about 6 hours or less, about 5 hours or less, about 4 hours or less, about 3 hours or less, about 2 hours or less, about 1.5 hour or less, about 1 hour or less, about 50 minutes or less, about 45 minutes or less, about 40 minutes or less, about 35 minutes or less, or about 30 minutes or less post administration.

In some examples, the biocompatible fluorophore is soluble in aqueous solvents. In some embodiments, the fluorophore can comprise at least one ion under physiological conditions for improving aqueous solubility. The term "ion," as used herein, refers to any molecule, portion of a molecule, cluster of molecules, molecular complex, moiety, or atom that contains a charge (positive, negative, or both at the same time within one molecule, cluster of molecules, molecular complex, or moiety (*e.g.*, Zwitterions)) or that can be made to contain a charge. Methods for producing a charge in a molecule, portion of a molecule, cluster of molecules, molecular complex, moiety, or atom are disclosed herein and can be accomplished by methods known in the art, *e.g.*, protonation, deprotonation, oxidation, reduction, alkylation, acetylation, esterification, deesterification, hydrolysis, etc. Exemplary ionic groups include, but are not limited to, sulfonic acid, sulfuric acid, boric acid, carboxylic acid, phosphonic acids, phosphoric acid, thioacetic acid, thiols, thiosulphate, oxalic acids, nitro group, amino acids, amine, ammonium group, alkoxide, salts thereof, and mixtures thereof. In some embodiments, the fluorophore is a zwitterion.

In some aspects, the positive/negative charge density, the charge distribution, and/or the net charge of the fluorophore can be used to improve the aqueous stability of the fluorophores. For example, the side chains of the fluorophores can be modified with a charged group such as a quaternary ammonium group or sulfonate group to improve the aqueous stability of the fluorophores.

As discussed herein, the fluorophores can exhibit relatively high solubility in water. As such, the fluorophore can have a log D that is less than 1, such as 0.5 or less or 0.1 or less. Log D, as used herein, refers to the ratio of the sum of the concentrations of all forms of the fluorophore (ionized plus un-ionized) in each of two phases, an octanol phase and a water phase. For measurements of distribution coefficient, the pH of the aqueous phase is buffered to 7.4 such that the pH is not significantly perturbed by the introduction of the compound. The logarithm of the ratio of the sum of concentrations of the solute's various forms in one solvent, to the sum of the concentrations of its forms in the other solvent is called Log D:

$$\log D_{\text{oct/wat}} = \log([\text{solute}]_{\text{octanol}} / ([\text{solute}]_{\text{ionized water}} + [\text{solute}]_{\text{neutral water}}))$$

**Methods of making**

Methods of making surgical articles are described herein. The fluorophore is physically affixed to the surgical article by one or more physical forces selected from hydrogen bonding, hydrophobic interaction or ionic forces or chemically affixed to the surgical article by covalent bonds. The fluorophore can be substantially throughout or at least on a portion of the surgical article, for example on the outside or on terminal portion of the surgical article. In some embodiments, the fluorophore can be dispersed in a polymeric film which is then used to coat the surgical article.

The fluorophore can be physically or chemically affixed to a surgical article, wherein the surgical article is formed

from fibers. In some embodiments, the fluorophore can comprise an acid group, such as a sulfonic or a carboxylic acid salt functional group. Fibers which can develop a positive charge can act as a driving force for agent diffusion and migration of the fluorophore into the fiber. Fibers such as wool, silk, and other protein fibers, nylon, and certain modified synthetics can develop a positive charge in the presence of an acid. In some embodiments, methods of affixing the fluorophore to a fiber can include contacting the fiber with an acid solution of the fluorophore, wherein the fluorophore comprises an acid group.

In some embodiments, the fluorophore (for example, the NIR agent) can comprise a long, narrow, and flat in molecular structure, which allows them to readily enter a fibrous structure, for example a cellulose structure. Due to the fluorophore's size and shape, they can in some embodiments interact with the fibers in such a way as to provide good fiber affinity. Since charge development is not a primary consideration in diffusion of some fluorophore onto the fiber, the agent can be applied from a solutions in which the fiber is more stable and more likely to swell. In some embodiments, the solvent system can be neutral or basic. In some embodiments, methods of affixing the fluorophore to the surgical article can include establishing a solvent system capable of swelling the fiber, contacting the fiber with the solvent system for a time adequate to swell the fiber, and contacting the swollen fiber with a solution of the biocompatible fluorophore.

In some embodiments, the fluorophore can comprise a reactive functional group capable of covalent bond formation with the fiber. The reactive functional group can be selected for incorporation into the fluorophore which will react readily with the fiber after diffusion into the structure but which will not decompose (such as by hydrolysis) in the solvent used in agent application. Acidic or basic conditions may be used to increase the reaction rate of the fluorophore with the fiber. Surgical articles comprising cellulosic, protein, and nylon fibers comprise reactive groups that can be covalently bonded to the fluorophore. In some embodiments, methods of affixing the fluorophore to the surgical article can include contacting the fiber with a solution of the fluorophore, wherein the fluorophore comprises a reactive group that can react with the fiber.

In some embodiments, the fluorophore can comprise a basic and/or cationic group that can migrate toward negative charges inside fibers within the surgical article. Cellulosic, protein, nylon, acrylic, and specially modified synthetic fibers comprise or can be made to negatively charged groups.

Other methods known in the art for incorporation a dye into a fiber include the thermosol process, the exhaust process, and the continuous process are well-known dyeing processes. Any of these methods can be used for affixing the fluorophore to the surgical article. The method can include incorporating the fluorophore into the fiber before it is formed into a surgical article. In some embodiments, fibers comprising the fluorophore can be woven into a surgical article. In some embodiments, fibers comprising the fluorophore can be formed into a non-woven surgical article.

The fluorophore can be affixed to a plastic surgical article by incorporating (e.g., by means of compounding) the agent directly into the polymeric materials from which the article is prepared. Such direct incorporation method can result in the fluorophore being dispersed substantially throughout the molded article. In some embodiments, the fluorophore can be affixed to a portion of the surgical article as described in US Patent Application No. 2009/0089942. The method can include providing a plastic article comprising a polymer, contacting at least a portion of the surface of said plastic article with a treatment composition comprising at least one agent and water, maintaining said portion of said plastic article in contact with said treatment composition for a period of time, removing the treated plastic article from contact with said treatment composition, and rinsing the treated plastic article with water to remove excess agent.

The fluorophore can be affixed to a metal or glass surgical article directly or indirectly. Indirectly coating the surgical article can include forming a polymerized film on the surgical article and dispersing the fluorophore into the polymerized film.

In some embodiments, the fluorophore (for example, the NIR agent) can be incorporated into a core-shell nanoparticle. The core-shell nanoparticle offer many advantages for example, they can be made such that they are non-toxic, have excellent photophysical properties, high biocompatibility, and excellent renal clearance. The fluorophore can be covalently linked, encapsulated, absorbed, or dispersed within the core-shell nanoparticle. In some examples, the core-shell nanoparticles can comprise a fluorescent core and a shell such as a silica shell. The core-shell nanoparticle can comprise a surface coating that can be physically adsorbed or covalently bonded to the surgical article.

## Methods of Using

Methods of detecting the disclosed surgical articles in the body of a subject are provided. In some examples, the method involves detecting surgical articles that are inadvertently left within the body cavity of the subject. The detection of the surgical article inadvertently left within the body cavity can occur prior to or after closing the body cavity after a surgical procedure.

The method for detecting the surgical article includes illuminating a first portion of the subject with an excitation light source and observing the fluorescent signal (such as a near infrared or a visible fluorescent signal) in the subject. The fluorescent signal can be displayed on a computing device. The presence of an increased fluorescent signal, relative to a background staining intensity, indicates that the article is in the subject.

## Examples

The following examples are put forth so as to provide those of ordinary skill in the art with a complete disclosure and description of how the compounds, compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Example 1: Synthesis, Evaluation, and Incorporation of the NIR Agents into Surgical Sponges

*Synthesis of NIR agents:* A family of NIR heptamethine carbocyanine compounds and a family of squarylium compounds can be synthesized as shown in Schemes 1 and 2. The compounds in this example fluoresce above 800 nm and contains conjugatable functional groups that are compatible with fibers that are used to make surgical sponges.

As outlined in Scheme 1, the treatment of cycloalkene, **1** with PhNMeCHO and $POCl_3$, followed by $K_2CO_3$ in $H_2O$ and $PhNH_2$.HCl give the Vilsmeyer-Haack reagent, **2** in good yield. Reagent **2** reacts with various indolium salts to produce the heptamethine carbocyanines, **3** functionalized with groups that can be conjugated to the polymers/fibers in the surgical sponge. These heptamethine cyanines display excellent structural and photophysical stability in serum. Furthermore, the relative Z, X and R groups listed in Scheme 1 all provide synthetic routes for covalent conjugation to the surface of polymers/fibers in the surgical sponge while also increasing aqueous solubility of the compounds.

Using the salts shown in Scheme 1, the squarylium compounds in Scheme 2 can be synthesized as depicted to generate a different class of compounds as surgical sponge tags. The compounds shown in Schemes 1 and 2 fluoresce in the 800 nm region of the electromagnetic spectrum. The squarylium compounds display excellent fluorescence quantum yield and therefore can lower the limit of detection for the marked sponges.

Using similar chemistry, as outlined in Schemes 1 and 2, NIR agents with other heterocyclic ring systems can be prepared, for example, compounds of higher wavelengths or with additional functional groups. The characteristics of these NIR agents make them effective for intra-body detection, specifically the absorbance ($A_{max}$), fluorescence ($A_{em}$), the Stokes' shift, and the high quantum yield of these compounds, makes them suitable for use in detecting surgical sponges *in vivo.* These compounds are also non-toxic and have high elimination rates when injected *in vivo.*

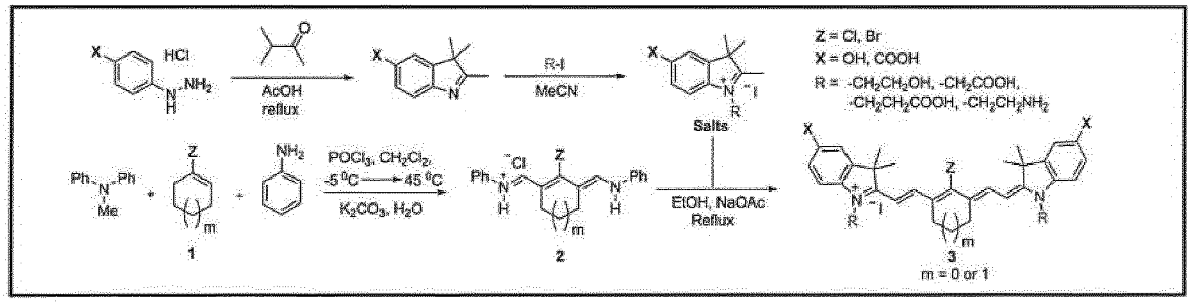


**Scheme 1.** Synthesis of NIR carbocyanine compounds

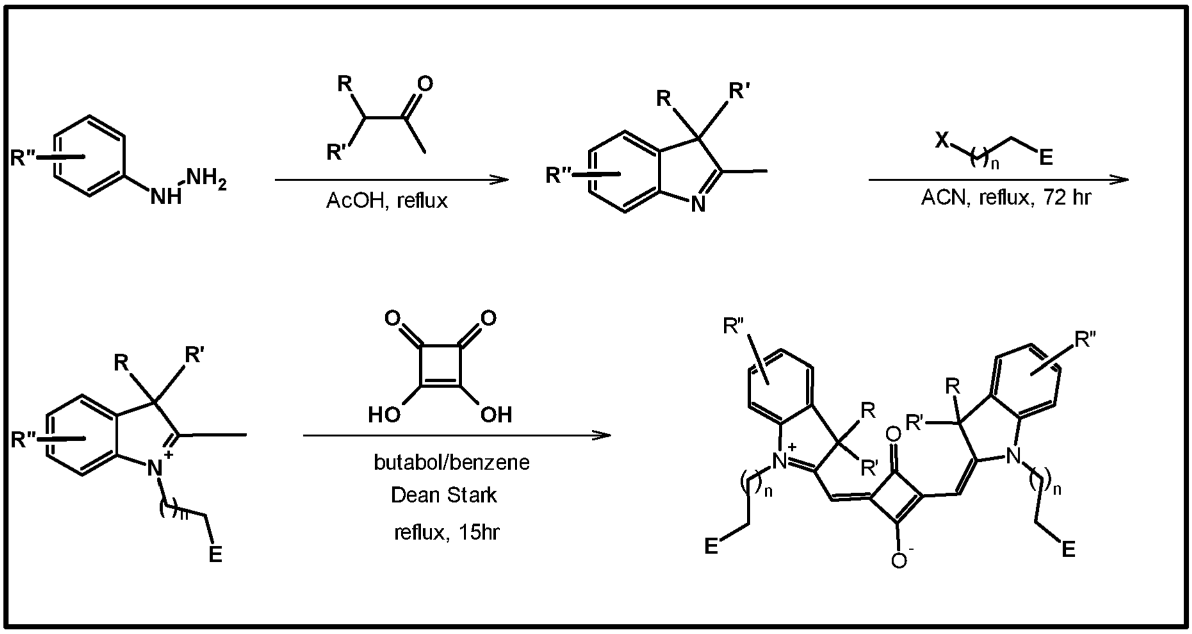


**Scheme 2.** Synthesis of stable NIR squarylium compounds. R, R', R", and E can be as described herein.

A family of NIR nile blue compounds can be synthesized as shown in Scheme 3. The compounds fluoresce above 700 nm and contains conjugatable functional groups that are compatible with fibers that are used to make surgical sponges.

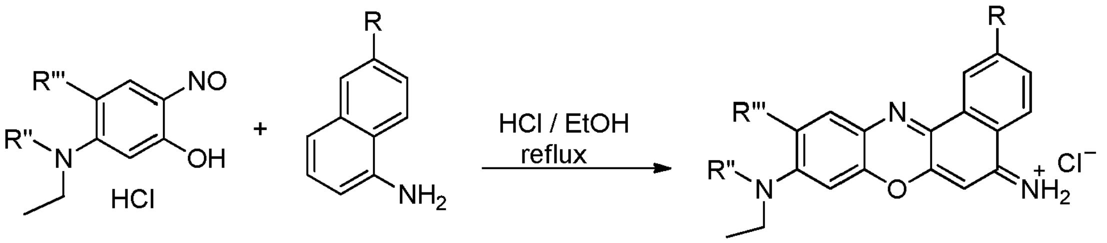


R = OH, $NH_2$, $OCH_2$-$CH_2$-$CH_2$-R'
where as R' = OH, COOH, $NH_2$;
R" = H, Me, Et, $CH_2CH_2COOH$;
R"' = H, Me, Br

**Scheme 3.** Synthesis of NIR nile blue compounds

*Evaluation of the analytical properties of the NIR agents:* The optical properties of the synthesized NIR agents, including absorbance, fluorescence, molar absorptivity, quantum yield and solvatochromic/hydrophobicity can be determined. Absorption measurements are collected with a Perkin-Elmer Lambda UVNIS/NIR (Lambda 50) Spectrophotometer (Norwalk, CT) and to ensure clarity in optical measurements, they are carried out in 1 cm quartz cuvettes. Laser Induced Fluorescence (UF) emission spectra were acquired using a K2 Spectrofluorometer (ISS, Champaign, IL) equipped with a R298 Hamamatsu Photomultiplier Tube (Bridgewater, NJ) with excitation achieved using laser excitation (Laser Max, Rochester, NY) at the appropriate wavelength corresponding to the particular compound. Slit widths are set to 2 mm and integration time of 3 s. Optical measurements are done at 37°C in 100% fetal bovine serum (FBS) buffered with 50 mM HEPES, pH 7.4. The hydrophobic characteristics of the cyanine compounds are evaluated by acquiring absorbance and emission spectra of the compounds in varying ratios of methanol-nanopure grade water mixture (in the range of 0-100% methanol/water) until there are no further discernible spectral changes, at which point

the spectra overlap as percent methanol increased.

*Incorporation of NIR agents in surgical sponge:* The NIR agents can be entrapped into surgical sponges to produce NIR-marked surgical sponges. The method of entrapping the NIR agents includes determining an organic solvent composition that swells the polymeric fiber to at least twice its original size in about 5-24 hours. Slow swelling is used to prevent damage to the fiber's mechanical integrity. The chosen solvent mixture is relatively volatile.

Once the solvent system and agent are selected, a 10 mM solution of the agent in the solvent system is slowly added to boiling water containing a fully immersed surgical sponge until a desired final concentration of agent in the sponge is achieved. The sponge is washed thoroughly with hot water and dried. The changes in the fluorescence intensity of the sponges are then analyzed, which correspond to the migration of the NIR agent into the material.

**Example 2: Indocyanine Green in Surgical Sponges**

*Method:* The following indocyanine Green (ICG) compound/$H_2O$ solutions were prepared: 1 mM solution, 1 μM solution, 1 nM solution, $10^{-5}$ M solution, and control (only water). The compound solutions were incorporated into surgical sponges as described in Example 1. The sponge products used included laparotomy pads (lap pads, several layers of gauze folded into a square), Raytec sponge (X-ray detectable sponge), gauze, and cottonoids (surgical patties).

The sponges were irradiated with a 690 nm laser and photographed using a NIR night vision monocular and an iPhone 3GS. These initial experiments indicated that the best concentrations to use were the 1 mM and 1 μM compound solutions. The control laparotomy pad and the pad treated with $10^{-5}$ M compound/water solution were soaked in pig's blood and observed for excitation.

A faux-abdomen was constructed using pig intestines, spleen, and blood. The sponges were then randomly placed within the faux abdomen to soak up blood and mimic an abdominal surgery scenario. The compound solutions were incorporated into surgical sponges as described in Example 1. The sponge products used include laparotomy pads (15 cm x 15 cm), Raytec sponge (X-ray detectable sponge), Curity gauze sponges (4 cm x 4 cm), and Codman cottonoids (surgical patties; 0.46 cm x 7.62 cm and 0.64 cm x 0.64 cm).

*Results:* As shown in Figure 1, as the concentration of the ICG compound solution increased, there was an increase in the fluorescence from the untreated lap pads to the lap pad treated with 1 nM up to 1 mM ICG sample.

As shown in Figures 2-6, there was an increase in the fluorescence of the test samples (treated with ICG compound) over the control, when placed in a faux abdomen.

**Example 3: *In vivo* Experiments**

*Method:* A stray female dog (Doberman Pincher) underwent an ovariohysterectomy procedure. After making an incision in the dog's abdomen, a standard 4x4 surgical gauze was placed inside the surgical site and allowed to become saturated with blood. A 780 nm laser was focused on a portion of the gauze and the area photographed and observed for excitation. This experiment was repeated using a sponge soaked in 1mM ICG dye solution for 1 hr and a sponge soaked in 1mM ICG dye solution for 24 hrs. Prior to *in vivo* use, the sponges first underwent sterilization: Steam sterilization at 350°F for 20 min and gas sterilization in ethylene oxide for 12-24 hr. The ICG sponges successfully survived both sterilization procedures.

Blood samples were taken from the dog to analyze, if any, NIR dye leaching from the sponges. The blood samples were extracted from the canine at: 0 hr (before sponge placement), 1 hr (post sponge placement), and 4 hrs (post sponge placement).

*Results:* As shown in Figure 7, there was a significant increase in the fluorescence of the sponge treated with 1 mM ICG solution compared to the untreated sponge. There was also an increase in florescence observed for the sponge soaked in 1mM ICG dye solution for 24 hrs compared to the sponge soaked in 1mM ICG dye solution for 1 hr.

As shown in Figure 8, the control canine blood sample, spiked with 0.1 mM solution of ICG in DMSO, fluoresced at ~815 nm. The test canine blood sample (containing blood and DMSO in a 1:3 ratio), at 1 hr and 4 hours after sponge placement, showed no fluorescence, which is indicative that no ICG leaching was detected.

**Example 4: Indocyanine Green in Plastic Articles**

*Method:* A thermoplastic that melts at 62°C (143°F) was placed into boiling water until malleable. The plastic was then soaked in a NIR dye solution and molded into a flat surface. The dye solution contained a 1 mM hydrophobic NIR dye. This hydrophobic dye was found to have increased fluorescence when compared to ICG, and because the dye is fixed within the plastic, there is no concern of leaching. The thermoplastic was then cooled and allowed to harden. A control plastic sample (not soaked in NIR dye solution) and a test plastic sample (soaked in NIR dye solution) were placed in a bath of porcine blood and observed for excitation.

*Results:* As shown in Figure 9, there was a significant increase in the fluorescence for the test plastic samples

compared to the control.

**Example 5: Indocyanine Green in Metal Articles**

*Method:* Portions of metal samples (including a hemostat and suture needles) were treated with a solution containing 1 mM ICG. The ICG was combined with a hypoallergenic fixing agent and applied to the metal surfaces and cured. The control metal samples and test metal samples were then placed in a bath of porcine blood or a porcine spleen and observed for excitation.

*Results:* As shown in Figures 10 and 11, there was a significant increase in the fluorescence for the test metal samples compared to the control.

## Claims

1. A surgical article comprising a material incorporating a fluorophore selected from a near infrared (NIR) agent or a fluorescent protein, wherein the NIR agent has a maximum absorption at a wavelength of from 650 nm to 1,000 nm, wherein the fluorophore is affixed to the material covalently, hydrophobically, ionically, by hydrogen bond, or combinations thereof.

2. The article of claim 1, wherein the surgical article is capable of absorbing fluids within a body.

3. The article of any one of claims 1 to 2, wherein the material is selected from the group consisting of a fabric, a paper, a plastic material, wood, a metal, glass, or combinations thereof, optionally wherein the material is a fabric formed from a natural fiber, a synthetic fiber, or combinations thereof, optionally wherein the material comprises a fiber selected from the group consisting of cellulose, protein, nylon, triacetate, polyester, and combinations thereof.

4. The article of any one of claims 1 to 3, wherein the surgical article is selected from the group consisting of a laparotomy pad, a sponge, a gauze, a cottonoid, a surgical packing, a needle, a surgical tape, a surgical retractor, a clip, a suture thread, a staple, a knife blade, a safety pin, a scalpel, a clamp, a scissors, a hemostat, a tweezer, a forcep, a suction tip or tube, a scope, an ultrasound tissue disruptor, an asepto bulb, a central line guide wire, a catheter, and combinations thereof.

5. The article of any one of claims 1 to 4, wherein the near infrared agent is selected from the group consisting of a cyanine compound, a coumarin compound, a squarylium compound, a 4,4-difluoro-4-bora-3a,4a-diaza-s-indacene (BODIPY) compound, an oxazine compound, a rhodamine compound, a phthalocyanine, a porphyrin, a derivative thereof, and combinations thereof.

6. The article of any one of claims 1 to 5, wherein the near infrared agent comprises at least one charged group under physiological conditions or is synthesized as a charged compound, wherein the charged group is selected from the group consisting of sulfonic acid, sulfuric acid, boric acid, carboxylic acid, phosphonic acids, phosphoric acid, thioacetic acid, thiols, thiosulphate, oxalic acids, nitro group, amino acids, amine, ammonium group, alkoxide, salts thereof, and mixtures thereof.

7. The article of any one of claims 1 to 5, wherein the near infrared agent includes a structure defined by Formula I, or a salt or a hydrate thereof,

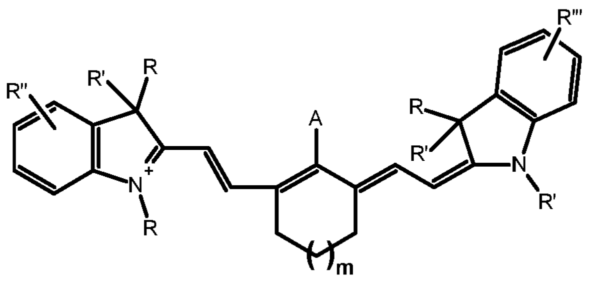

Formula I,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and

A, R" and R‴ are independently selected from the group consisting of hydrogen; halogen, alkyl, $-OR^1$; $-NR^1R^2$; $-NR^1R^2R^3$; $-NO_2$; $-CF_3$; -CN; $-C_2R'$; -SR'; - $N_3$; C(=O) $R^1$; $-C(=O)OR^1$; $-OC(=O)R^1$; $-O(CR^1R^2)_rC(=O)R^1$; $-C(=O)NR^1R^2$; - $NR^1C(=O)R^2$; $-O(CR^1R^2)_rNR^2C(=O)R^1$; $-O(CR^1R^2)_rNR^2SO_2R^1$; $-OC(=O)NR^1R^2$; - $NR^1C(=O)OR^2$; $-SO_2R^1$; $-SO_2NR^1R^2$; and $-NR^1SO_2R^2$; wherein $R^1$, $R^2$, and $R^3$ are individually hydrogen; substituted or unsubstituted alkyl; alkenyl; alkynyl; cycloalkyl; heterocyclyl; cycloalkenyl, heterocycloalkenyl, aryl, heteroaryl, arylalkyl, heteroalkyls, aryl, alkylaryl, alkylheteroaryl; and

wherein m is 0, 1, 2, or 3, and r is an integer from 1 to 6.

**8.** The article of any one of claims 1 to 6, wherein the near infrared agent includes a structure defined by Formula II, or a salt or a hydrate thereof,

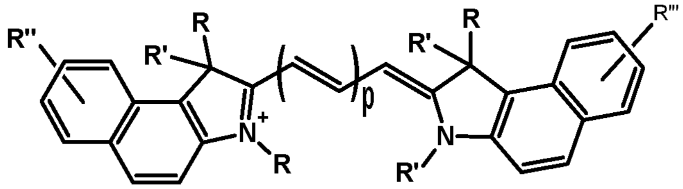

Formula II,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and

R" and R‴ are independently selected from the group consisting of hydrogen; halogen, alkyl, $-OR^1$; $-NR^1R^2$; $-NR^1R^2R^3$; $-NO_2$; $-CF_3$; -CN; $-C_2R'$; -SR'; $-N_3$; C(=O) $R^1$; $-C(=O)OR^1$; $-OC(=O)R^1$; $-O(CR^1R^2)_rC(=O)R^1$; $-C(=O)NR^1R^2$; $-NR^1C(=O)R^2$; - $O(CR^1R^2)_rNR^2C(=O)R^1$; $-O(CR^1R^2)_rNR^2SO_2R^1$; $-OC(=O)NR^1R^2$; $-NR^1C(=O)OR^2$; - $SO_2R^1$; $-SO_2NR^1R^2$; and $-NR^1SO_2R^2$; wherein $R^1$, $R^2$, and $R^3$ are individually hydrogen; substituted or unsubstituted alkyl; alkenyl; alkynyl; cycloalkyl; heterocyclyl; cycloalkenyl, heterocycloalkenyl, aryl, heteroaryl, arylalkyl, heteroalkyls, aryl, alkylaryl, alkylheteroaryl; and

wherein p is an integer from 0 to 3, and r is an integer from 1 to 6.

**9.** The article of any one of claims 1 to 5, wherein the near infrared agent includes a structure defined by Formula IV, or a salt or a hydrate thereof,

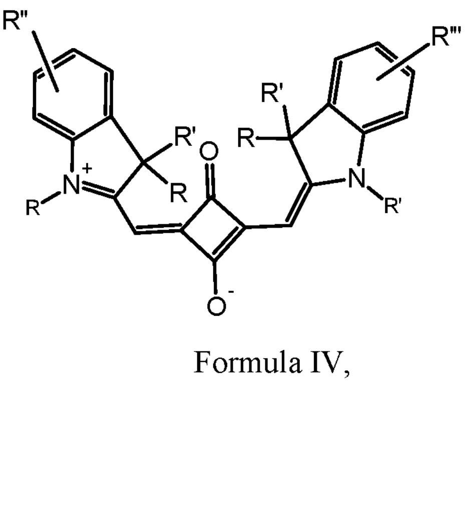

Formula IV,

wherein R and R' are independently selected from the group consisting of hydrogen; substituted or unsubstituted alkyl; substituted or unsubstituted cycloalkyl; substituted or unsubstituted alkenyl; substituted or unsubstituted alkynyl; substituted or unsubstituted heterocyclyl; substituted or unsubstituted cycloalkenyl; substituted or unsubstituted heterocycloalkenyl; substituted or unsubstituted aryl; substituted or unsubstituted heteroaryl; substituted or unsubstituted heteroalkyl; substituted or unsubstituted alkylaryl; substituted or unsubstituted alkylheteroaryl; and

R" and R‴ are independently selected from the group consisting of hydrogen; halogen; alkyl, $-OR^1$; $-NR^1R^2$; ;$-NO_2$; $-CF_3$; -CN; $-C_2R^1$; $-SR^1$; $-N_3$; $C(=O)R^1$; - $C(=O)OR^1$; $-OC(=O)R^1$; $-O(CR^1R^2)_rC(=O)R^1$; $-C(=O)NR^1R^2$; $-NR^1C(=O)R^2$; - $O(CR^1R^2)_rNR^2C(=O)R^1$; $-O(CR^1R^2)_rNR^2SO_2R^1$; $-OC(=O)NR^1R^2$; $-NR^1C(=O)OR^2$; - $SO_2R^1$; $-SO_2NR^1R^2$; and $-NR^1SO_2R^2$; wherein $R^1$ and $R^2$ are individually hydrogen; substituted or unsubstituted alkyl; substituted or unsubstituted alkenyl; substituted or unsubstituted alkynyl; substituted or unsubstituted cycloalkyl; substituted or unsubstituted heterocyclyl; substituted or unsubstituted cycloalkenyl, substituted or unsubstituted heterocycloalkenyl, substituted or unsubstituted aryl, substituted or unsubstituted heteroaryl, substituted or unsubstituted arylalkyl, substituted or unsubstituted heteroalkyl, or substituted or unsubstituted alkylheteroaryl; and

r is an integer from 1 to 6.

**10.** A method for detecting an article of any one of claims 1 to 9 in a subject, the method comprising,

illuminating a first portion of the subject with an excitation light source, and
observing a fluorescent signal in the subject,
wherein the presence of an increased fluorescent signal, relative to a background staining intensity, indicates that the article is in the subject.

**11.** The method of claim 10, further comprising displaying the fluorescent signal on a computing device.

**12.** A method of making the surgical article of claim 1 comprising,

affixing the near infrared agent or fluorescent protein to a plurality of biocompatible fibers; and
assembling the biocompatible fibers into the surgical article.

**13.** The method of claim 12, wherein the fluorophore comprises a group reactive with a functional group present in the biocompatible fiber, optionally wherein the reactive group includes an amino group, a hydroxyl group, an alkenyl group, an alkoxy group, a sulfonyl group, a carboxylic acid group, or combinations thereof.

**14.** The method of claim 13 comprising:

establishing a solvent system capable of swelling the biocompatible fibers,
contacting the biocompatible fibers with the solvent system for a time adequate to swell the biocompatible fibers;
contacting the swollen biocompatible fibers with a solution of the near infrared agent or the fluorescent protein; and
forming the biocompatible fibers into the surgical article.

## Patentansprüche

**1.** Chirurgischer Gegenstand, umfassend ein Material, das einen Fluorophor ausgewählt aus einem Nahinfrarot(NIR)-Mittel und einem Fluoreszenzprotein enthält, wobei das NIR-Mittel eine maximale Absorption bei einer Wellenlänge von 650 nm bis 1.000 nm aufweist, wobei der Fluorophor kovalent, hydrophob, ionisch, durch Wasserstoffbrücken oder Kombinationen davon an dem Material befestigt ist.

**2.** Gegenstand gemäß Anspruch 1, wobei der chirurgische Gegenstand fähig ist, Fluide in einem Körper zu absorbieren.

**3.** Gegenstand gemäß einem der Ansprüche 1 bis 2, wobei das Material ausgewählt ist aus der Gruppe bestehend aus einem Gewebe, einem Papier, einem Kunststoffmaterial, Holz, einem Metall, Glas und Kombinationen davon, wobei das Material gegebenenfalls ein Gewebe ist, das aus einer natürlichen Faser, einer synthetischen Faser oder Kombinationen davon gebildet ist, wobei das Material gegebenenfalls eine Faser ausgewählt aus der Gruppe bestehend aus Cellulose, Protein, Nylon, Triacetat, Polyester und Kombinationen davon umfasst.

4. Gegenstand gemäß einem der Ansprüche 1 bis 3, wobei der chirurgische Gegenstand ausgewählt ist aus der Gruppe bestehend aus einem Laparotomie-Pad, einem Schwamm, einer Gaze, einem Cottonoid, einer chirurgischen Packung, einer Nadel, einem chirurgischen Band, einem chirurgischen Retraktor, einem Clip, einem Nahtfaden, einer Klammer, einer Messerklinge, einem Sicherheitsstift, einem Skalpell, einer Klemme, einer Schere, einer Gefäßklemme, einer Pinzette, einer Zange, einer/einem Saugspitze oder -rohr, einer Scope-Vorrichtung, einem Ultraschall-Gewebedisruptor, einer Asepto-Birne, einem Zentralvenenkatheter-Führungsdraht, einem Katheter und Kombinationen davon.

5. Gegenstand gemäß einem der Ansprüche 1 bis 4, wobei das Nahinfrarotmittel ausgewählt ist aus der Gruppe bestehend aus einer Cyaninverbindung, einer Cumarinverbindung, einer Squaryliumverbindung, einer 4,4-Difluor-4-bora-3a,4a-diaza-s-indacen(BODIPY)-Verbindung, einer Oxazinverbindung, einer Rhodaminverbindung, einem Phthalocyanin, einem Porphyrin, einem Derivat davon und Kombinationen davon.

6. Gegenstand gemäß einem der Ansprüche 1 bis 5, wobei das Nahinfrarotmittel wenigstens eine unter physiologischen Bedingungen geladene Gruppe umfasst oder als geladene Verbindung synthetisiert ist, wobei die geladene Gruppe ausgewählt ist aus der Gruppe bestehend aus Sulfonsäure, Schwefelsäure, Borsäure, Carbonsäure, Phosphonsäuren, Phosphorsäure, Thioessigsäure, Thiolen, Thiosulfat, Oxalsäuren, Nitrogruppe, Aminosäuren, Amin, Ammoniumgruppe, Alkoxid, Salzen davon und Gemischen davon.

7. Gegenstand gemäß einem der Ansprüche 1 bis 5, wobei das Nahinfrarotmittel eine durch Formel I definierte Struktur oder ein Salz davon oder ein Hydrat davon enthält,

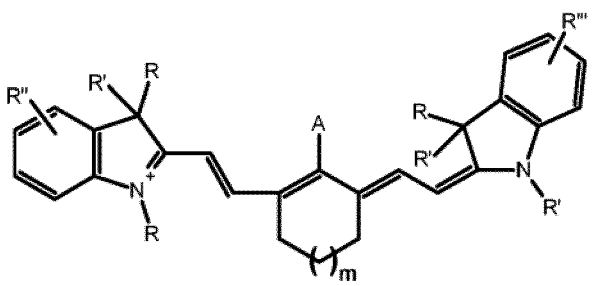

Formel I,

wobei R und R' unabhängig ausgewählt sind aus der Gruppe bestehend aus Wasserstoff; substituiertem oder unsubstituiertem Alkyl; substituiertem oder unsubstituiertem Cycloalkyl; substituiertem oder unsubstituiertem Alkenyl; substituiertem oder unsubstituiertem Alkinyl; substituiertem oder unsubstituiertem Heterocyclyl; substituiertem oder unsubstituiertem Cycloalkenyl; substituiertem oder unsubstituiertem Heterocycloalkenyl; substituiertem oder unsubstituiertem Aryl; substituiertem oder unsubstituiertem Heteroaryl; substituiertem oder unsubstituiertem Heteroalkyl; substituiertem oder unsubstituiertem Alkylaryl; substituiertem oder unsubstituiertem Alkylheteroaryl; und

A, R" und R‴ unabhängig ausgewählt sind aus der Gruppe bestehend aus Wasserstoff; Halogen; Alkyl; $-OR^1$; $-NR^1R^2$; $-NR^1R^2R^3$; $-NO_2$; $-CF_3$; -CN; $-C_2R'$; -SR'; $-N_3$; $C(=O)R^1$; $-C(=O)OR^1$; $-OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; $-O(CR^1R^2)_rNR^2SO_2R^1$; $-OC(=O)NR^1R^2$; $-NR^1C(=O)OR^2$; $-SO_2R^1$; $-SO_2NR^1R^2$; und $-NR^1SO_2R^2$; wobei $R^1$, $R^2$ und $R^3$ unabhängig Wasserstoff, substituiertes oder unsubstituiertes Alkyl; Alkenyl; Alkinyl; Cycloalkyl; Heterocyclyl; Cycloalkenyl, Heterocycloalkenyl, Aryl, Heteroaryl, Arylalkyl, Heteroalkyle, Aryl, Alkylaryl, Alkylheteroaryl sind; und

wobei m 0, 1, 2 oder 3 ist und r eine ganze Zahl von 1 bis 6 ist.

8. Gegenstand gemäß einem der Ansprüche 1 bis 6, wobei das Nahinfrarotmittel eine durch Formel II definierte Struktur oder ein Salz davon oder ein Hydrat davon enthält,

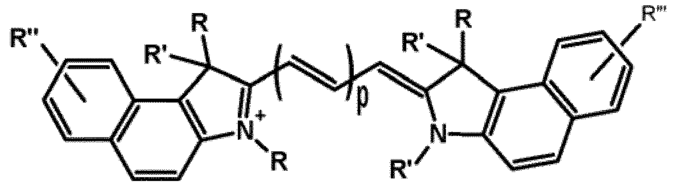

Formel II,

wobei R und R' unabhängig ausgewählt sind aus der Gruppe bestehend aus Wasserstoff; substituiertem oder unsubstituiertem Alkyl; substituiertem oder unsubstituiertem Cycloalkyl; substituiertem oder unsubstituiertem Alkenyl; substituiertem oder unsubstituiertem Alkinyl; substituiertem oder unsubstituiertem Heterocyclyl; substituiertem oder unsubstituiertem Cycloalkenyl; substituiertem oder unsubstituiertem Heterocycloalkenyl; substituiertem oder unsubstituiertem Aryl; substituiertem oder unsubstituiertem Heteroaryl; substituiertem oder unsubstituiertem Heteroalkyl; substituiertem oder unsubstituiertem Alkylaryl; substituiertem oder unsubstituiertem Alkylheteroaryl; und

R" und R‴ unabhängig ausgewählt sind aus der Gruppe bestehend aus Wasserstoff; Halogen; Alkyl; $-OR^1$; $-NR^1R^2$; $-NR^1R^2R^3$; $-NO_2$; $-CF_3$; -CN; $-C_2R'$; -SR'; $-N_3$; $C(=O)R^1$; $-C(=O)OR^1$; $-OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; $-O(CR^1R^2)_rNR^2SO_2R^1$; $-OC(=O)NR^1R^2$; $-NR^1C(=O)OR^2$; $-SO_2R^1$; $-SO_2NR^1R^2$; und $-NR^1SO_2R^2$; wobei $R^1$, $R^2$ und $R^3$ unabhängig Wasserstoff, substituiertes oder unsubstituiertes Alkyl; Alkenyl; Alkinyl; Cycloalkyl; Heterocyclyl; Cycloalkenyl, Heterocycloalkenyl, Aryl, Heteroaryl, Arylalkyl, Heteroalkyle, Aryl, Alkylaryl, Alkylheteroaryl sind; und

wobei p eine ganze Zahl von 0 bis 3 ist und r eine ganze Zahl von 1 bis 6 ist.

**9.** Gegenstand gemäß einem der Ansprüche 1 bis 5, wobei das Nahinfrarotmittel eine durch Formel IV definierte Struktur oder ein Salz davon oder ein Hydrat davon enthält,

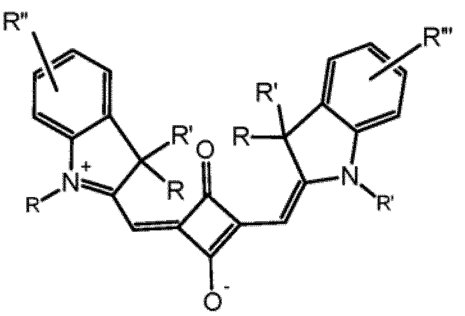

Formel II,

wobei R und R' unabhängig ausgewählt sind aus der Gruppe bestehend aus Wasserstoff; substituiertem oder unsubstituiertem Alkyl; substituiertem oder unsubstituiertem Cycloalkyl; substituiertem oder unsubstituiertem Alkenyl; substituiertem oder unsubstituiertem Alkinyl; substituiertem oder unsubstituiertem Heterocyclyl; substituiertem oder unsubstituiertem Cycloalkenyl; substituiertem oder unsubstituiertem Heterocycloalkenyl; substituiertem oder unsubstituiertem Aryl; substituiertem oder unsubstituiertem Heteroaryl; substituiertem oder unsubstituiertem Heteroalkyl; substituiertem oder unsubstituiertem Alkylaryl; substituiertem oder unsubstituiertem Alkylheteroaryl; und

R" und R‴ unabhängig ausgewählt sind aus der Gruppe bestehend aus Wasserstoff; Halogen; Alkyl; $-OR^1$; $-NR^1R^2$; $-NO_2$; $-CF_3$; -CN; $-C_2R^1$; $-SR^1$; $-N_3$; $C(=O)R^1$; $-C(=O)OR^1$; $-OC(=O)R^1$; $-O(CR^1R^2)_rC(=O)R^1$; $-C(=O)NR^1R^2$; $-NR^1C(=O)R^2$; $-O(CR^1R^2)_rNR^2C(=O)R^1$; $-O(CR^1R^2)_rNR^2SO_2R^1$; $-OC(=O)NR^1R^2$; $-NR^1C(=O)OR^2$; $-SO_2R^1$; $-SO_2NR^1R^2$; und $-NR^1SO_2R^2$; wobei $R^1$ und $R^2$ unabhängig Wasserstoff, substituiertes oder unsubstituiertes Alkyl; substituiertem oder unsubstituiertes Alkenyl; substituiertes oder unsubstituiertes Alkinyl; substituiertes oder unsubstituiertes Cycloalkyl; substituiertes oder unsubstituiertes Heterocyclyl; substituiertes oder unsubstituiertes Cycloalkenyl; substituiertes oder unsubstituiertes Heterocycloalkenyl; substituiertes oder unsubstituiertes Aryl; substituiertes oder unsubstituiertes Heteroaryl; substituiertes oder unsubstituiertes Arylalkyl; substituiertes oder unsubstituiertes Heteroalkyl; substituiertes oder unsubstituiertes Alkylheteroaryl sind; und
r eine ganze Zahl von 1 bis 6 ist.

**10.** Verfahren zum Nachweisen eines Gegenstands gemäß einem der Ansprüche 1 bis 9 in einem Subjekt, wobei das Verfahren umfasst:

Beleuchten eines ersten Teils des Subjekts mit einer Anregungslichtquelle und
Beobachten eines Fluoreszenzsignals in dem Subjekt,
wobei das Vorhandensein eines erhöhten Fluoreszenzsignals im Vergleich zu einer Hintergrund-Färbungsintensität anzeigt, dass sich der Gegenstand in dem Subjekt befindet.

**11.** Verfahren gemäß Anspruch 10, ferner umfassend Anzeigen des Fluoreszenzsignals auf einer Computervorrichtung.

**12.** Verfahren zur Herstellung des chirurgischen Gegenstands gemäß Anspruch 1, umfassend

Befestigen des Nahinfrarotmittels oder Fluoreszenzproteins an einer Vielzahl von biokompatiblen Fasern; und
Zusammensetzen der biokompatiblen Fasern zu dem chirurgischen Gegenstand.

**13.** Verfahren gemäß Anspruch 12, wobei der Fluorophor eine Gruppe umfasst, die mit einer in der biokompatiblen Faser vorhandenen funktionellen Gruppe reaktionsfähig ist, wobei die reaktionsfähige Gruppe gegebenenfalls eine Aminogruppe, eine Hydroxygruppe, eine Alkenylgruppe, eine Alkoxygruppe, eine Sulfonylgruppe, eine Carbonsäuregruppe oder Kombinationen davon enthält.

**14.** Verfahren gemäß Anspruch 13, umfassend:

Einrichten eines Lösungsmittelsystems, das zum Quellen der biokompatiblen Fasern fähig ist;
Inkontaktbringen der biokompatiblen Fasern mit dem Lösungsmittelsystem für einen Zeitraum, der zum Quellen der biokompatiblen Fasern geeignet ist;
Inkontaktbringen der gequollenen biokompatiblen Fasern mit einer Lösung des Nahinfrarotmittels oder des Fluoreszenzproteins; und
Formen der biokompatiblen Fasern zu dem chirurgischen Gegenstand.

## Revendications

**1.** Article chirurgical comprenant un matériau incorporant un fluorophore choisi parmi un agent infrarouge proche (NIR) ou une protéine fluorescente, l'agent NIR possédant une absorption maximale à une longueur d'onde allant de 650 nm à 1 000 nm, le fluorophore étant fixé au matériau de manière covalente, de manière hydrophobe, de manière ionique, par une liaison hydrogène, ou des combinaisons correspondantes.

**2.** Article selon la revendication 1, l'article chirurgical étant capable d'absorber des fluides à l'intérieur d'un corps.

**3.** Article selon l'une quelconque des revendications 1 à 2, le matériau étant choisi dans le groupe constitué par un tissu, un papier, une matière plastique, du bois, un métal, du verre ou des combinaisons correspondantes, éventuellement, le matériau étant un tissu formé à partir d'une fibre naturelle, d'une fibre synthétique ou des combinaisons correspondantes, éventuellement, le matériau comprenant une fibre choisie dans le groupe constitué par une cellulose, une protéine, du nylon, du triacétate, un polyester et des combinaisons correspondantes.

**4.** Article selon l'une quelconque des revendications 1 à 3, l'article chirurgical étant choisi dans le groupe constitué par un tampon de laparotomie, une éponge, une gaze, un coton-tige, un emballage chirurgical, une aiguille, un ruban chirurgical, un écarteur chirurgical, une pince, un fil de suture, une agrafe, une lame de couteau, une épingle de sûreté, un scalpel, une pince, des ciseaux, un hémostatique, une pince à épiler, un forceps, un embout ou un tube d'aspiration, un scope, un dislocateur de tissu à ultrasons, une poire aseptique, un fil de guidage de ligne central, un cathéter, et des combinaisons correspondantes.

**5.** Article selon l'une quelconque des revendications 1 à 4, l'agent infrarouge proche étant choisi dans le groupe constitué par un composé de cyanine, un composé de coumarine, un composé de squarylium, un composé de 4,4-difluoro-4-bora-3a,4a-diaza-s-indacène (BODIPY), un composé d'oxazine, un composé de rhodamine, une phtalocyanine, une porphyrine, un dérivé correspondant, et des combinaisons correspondantes.

**6.** Article selon l'une quelconque des revendications 1 à 5, l'agent infrarouge proche comprenant au moins un groupe chargé dans des conditions physiologiques ou étant synthétisé comme un composé chargé, le groupe chargé étant choisi dans le groupe constitué par l'acide sulfonique, l'acide sulfurique, l'acide borique, un acide carboxylique, les acides phosphoniques, l'acide phosphorique, l'acide thioacétique, les thiols, un thiosulfate, les acides oxaliques, le groupe nitro, les acides aminés, une amine, un groupe ammonium, un alcoxyde, des sels correspondants, et des mélanges correspondants.

**7.** Article selon l'une quelconque des revendications 1 à 5, l'agent infrarouge proche comprenant une structure définie par la formule I, ou un sel ou un hydrate correspondant,

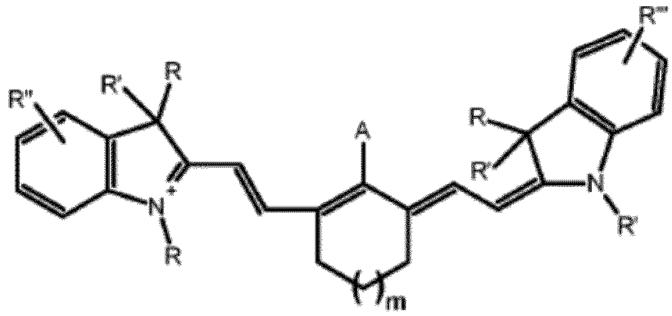

formule I,

R et R' étant indépendamment choisis dans le groupe constitué par hydrogène ; alkyle substitué ou non substitué ; cycloalkyle substitué ou non substitué ; alcényle substitué ou non substitué ; alcynyle substitué ou non substitué ; hétérocyclyle substitué ou non substitué ; cycloalcényle substitué ou non substitué ; hétérocycloalcényle substitué ou non substitué ; aryle substitué ou non substitué ; hétéroaryle substitué ou non substitué ; alkylaryle substitué ou non substitué ; alkylhétéroaryle substitué ou non substitué ; et

A, R'' et R''' étant indépendamment choisis dans le groupe constitué par hydrogène ; halogène, alkyle, $-OR^1$ ; $-NR^1R^2$ ; $-NR^1R^2R^3$ ; $-NO_2$ ; $-CF_3$ ; -CN ; $-C_2R'$ ; -SR' ; $-N_3$ ; $C(=O)R^1$ ; $-C(=O)OR^1$ ; $-OC(=O)R^1$ ; $-O(CR^1R^2)C(=O)R^1$ ; $-C(=O)NR^1R^2$ ; $-NR^1C(=O)R^2$ ; - $O(CR^1R^2)_rNR^2C(=O)R^1$ ; $-O(CR^1R^2)_rNR^2SO_2R^1$ ; - $OC(O)NR^1R^2$ ; $-NR^1C(=O)OR^2$ ; $-SO_2R^1$ ; $-SO_2NR^1R^2$ ; et - $NR^1SO_2R^2$ ; $R^1$, $R^2$, et $R^3$ étant individuellement hydrogène ; alkyle substitué ou non substitué ; alcényle ; alcynyle ; cycloalkyle ; hétérocyclyle ; cycloalcényle, hétérocycloalcényle, aryle, hétéroaryle, arylalkyle, des hétéroalkyles, aryle, alkylaryle, alkylhétéroaryle ; et

m étant 0, 1, 2 ou 3, et r étant un entier de 1 à 6.

**8.** Article selon l'une quelconque des revendications 1 à 6, l'agent infrarouge proche comprenant une structure définie par la formule II, ou un sel ou un hydrate correspondant,

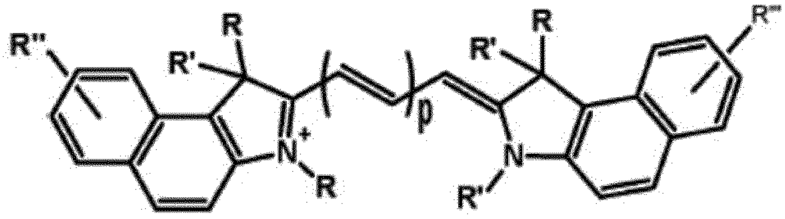

formule II,

R et R' étant indépendamment choisis dans le groupe constitué par hydrogène ; alkyle substitué ou non substitué ; cycloalkyle substitué ou non substitué ; alcényle substitué ou non substitué ; alcynyle substitué ou non substitué ; hétérocyclyle substitué ou non substitué ; cycloalcényle substitué ou non substitué ; hétérocycloalcényle substitué ou non substitué ; aryle substitué ou non substitué ; hétéroaryle substitué ou non substitué ; alkylaryle substitué ou non substitué ; alkylhétéroaryle substitué ou non substitué ; et

R'' et R''' étant indépendamment choisis dans le groupe constitué par hydrogène ; halogène, alkyle, $-OR^1$ ;

$-NR^1R^2$ ; $-NR^1R^2R^3$ ; $-NO_2$ ; $-CF_3$ ; -CN ; $-C_2R'$ ; -SR' ; $-N_3$ ; $C(=O)R^1$ ; $-C(=O)OR^1$ ; $-OC(=O)R^1$ : $-O(CR^1R^2)C(=O)R^1$ ; $-C(=O)NR^1R^2$ ; $-NR^1C(=O)R^2$ ; $-O(CR^1R^2)_rNR^2C(=O)R^1$ ; $-O(CR^1R^2)_rNR^2SO_2R^1$ ; $-OC(O)NR^1R^2$ ; $-NR^1C(=O)OR^2$ ; $-SO_2R^1$ ; $-SO_2NR^1R^2$ ; et $-NR^1SO_2R^2$ ; $R^1$, $R^2$, et $R^3$ étant individuellement hydrogène ; alkyle substitué ou non substitué ; alcényle ; alcynyle ; cycloalkyle ; hétérocyclyle ; cycloalcényle, hétérocycloalcényle, aryle, hétéroaryle, arylalkyle, des hétéroalkyles, aryle, alkylaryle, alkylhétéroaryle ; et p étant un entier de 0 à 3, et r étant un entier de 1 à 6.

**9.** Article selon l'une quelconque des revendications 1 à 5, l'agent infrarouge proche comprenant une structure définie par la formule IV, ou un sel ou un hydrate correspondant,

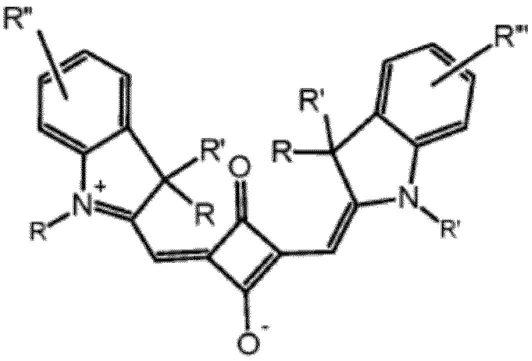

formule IV,

R et R' étant indépendamment choisis dans le groupe constitué par hydrogène ; alkyle substitué ou non substitué ; cycloalkyle substitué ou non substitué ; alcényle substitué ou non substitué ; alcynyle substitué ou non substitué ; hétérocyclyle substitué ou non substitué ; cycloalcényle substitué ou non substitué ; hétérocycloalcényle substitué ou non substitué ; aryle substitué ou non substitué ; hétéroaryle substitué ou non substitué ; alkylaryle substitué ou non substitué ; alkylhétéroaryle substitué ou non substitué ; et
R" et R‴ étant indépendamment choisis dans le groupe constitué par hydrogène ; halogène, alkyle, $-OR^1$ ; $-NR^1R^2$ ; $-NO_2$ ; $-CF_3$ ; -CN ; $-C_2R^1$ ; $-SR^1$ ; $-N_3$ ; $C(=O) R^1$ ; $-C(=O)OR^1$ ; $-OC(=O)R^1$ : $-O(CR^1R^2)_rC(=O)R^1$ ; $-C(=O)NR^1R^2$: $-NR^1C(=O)R^2$ ; $-O(CR^1R^2)_rNR^2C(=O)R^1$ ; $-O(CR^1R^2)_rNR^2SO_2R^1$ ; $-OC(O)NR^1R^2$ : $-NR^1C(=O)OR^2$ ; $-SO_2R^1$ ; $-SO_2NR^1R^2$ ; et $-NR^1SO_2R^2$ ; $R^1$ et $R^2$ étant individuellement hydrogène ; alkyle substitué ou non substitué ; alcényle substitué ou non substitué ; alcynyle substitué ou non substitué ; cycloalkyle substitué ou non substitué ; hétérocyclyle substitué ou non substitué ; cycloalcényle substitué ou non substitué ; hétérocycloalcényle substitué ou non substitué ; aryle substitué ou non substitué ; hétéroaryle substitué ou non substitué ; alkylaryle substitué ou non substitué ; hétéroaryle substitué ou non substitué ; alkylhétéroaryle substitué ou non substitué ; et
r étant un entier de 1 à 6.

**10.** Procédé pour la détection d'un article selon l'une quelconque des revendications 1 à 9 chez un sujet, le procédé comprenant,

l'illumination d'une première partie du sujet avec une source de lumière d'excitation, et
l'observation d'un signal fluorescent chez le sujet,
la présence d'un signal fluorescent augmenté, par rapport à une intensité de coloration de fond, indiquant que l'article est dans le sujet.

**11.** Procédé selon la revendication 10, comprenant en outre l'affichage du signal fluorescent sur un dispositif informatique.

**12.** Procédé de préparation de l'article chirurgical selon la revendication 1, comprenant

la fixation de l'agent infrarouge proche ou de la protéine fluorescente sur une pluralité de fibres biocompatibles ; et l'assemblage des fibres biocompatibles dans l'article chirurgical.

**13.** Procédé selon la revendication 12, le fluorophore comprenant un groupe réactif avec un groupe fonctionnel présent

dans la fibre biocompatible, éventuellement, le groupe réactif comprenant un groupe amino, un groupe hydroxyle, un groupe alcényle, un groupe alcoxy, un groupe sulfonyle, un groupe acide carboxylique ou des combinaisons correspondantes.

**14.** Procédé selon la revendication 13 comprenant :

l'établissement d'un système de solvant capable de gonfler les fibres biocompatibles,
la mise en contact des fibres biocompatibles avec le système de solvant pendant une durée adéquate pour gonfler les fibres biocompatibles ;
la mise en contact des fibres biocompatibles gonflées avec une solution de l'agent infrarouge proche ou de la protéine fluorescente ; et
la formation des fibres biocompatibles dans l'article chirurgical.

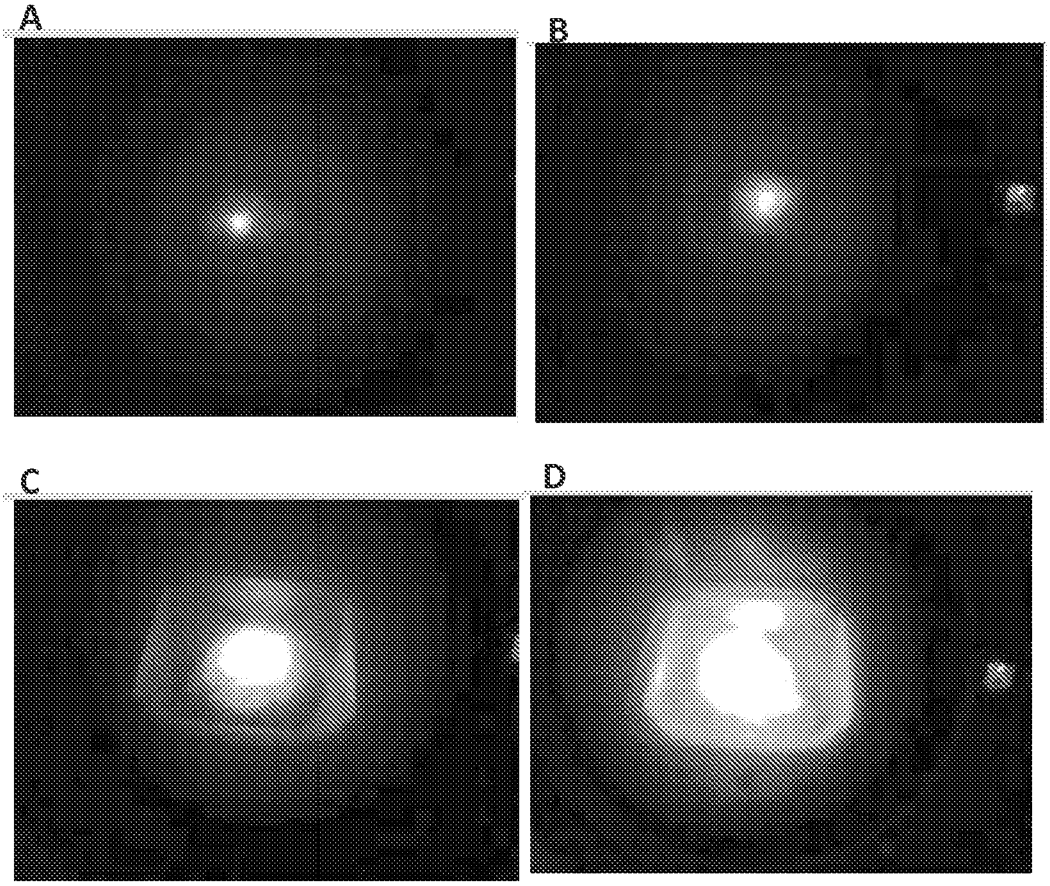


Figures 1A-1D

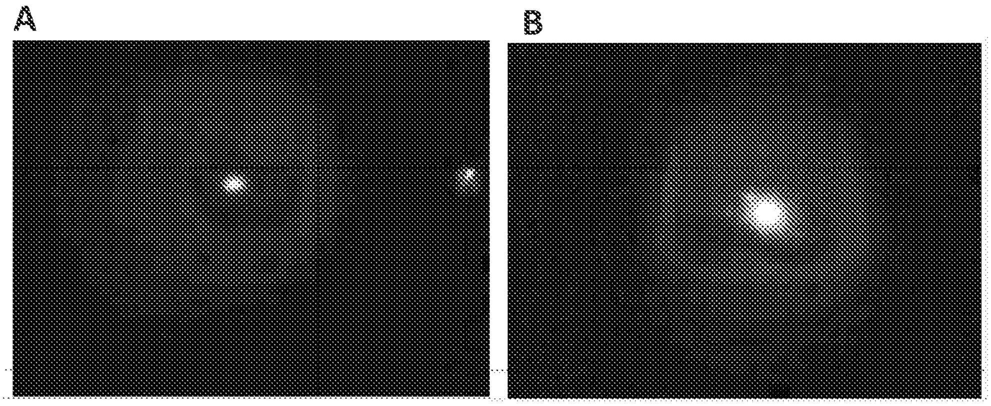


Figures 2A-2B

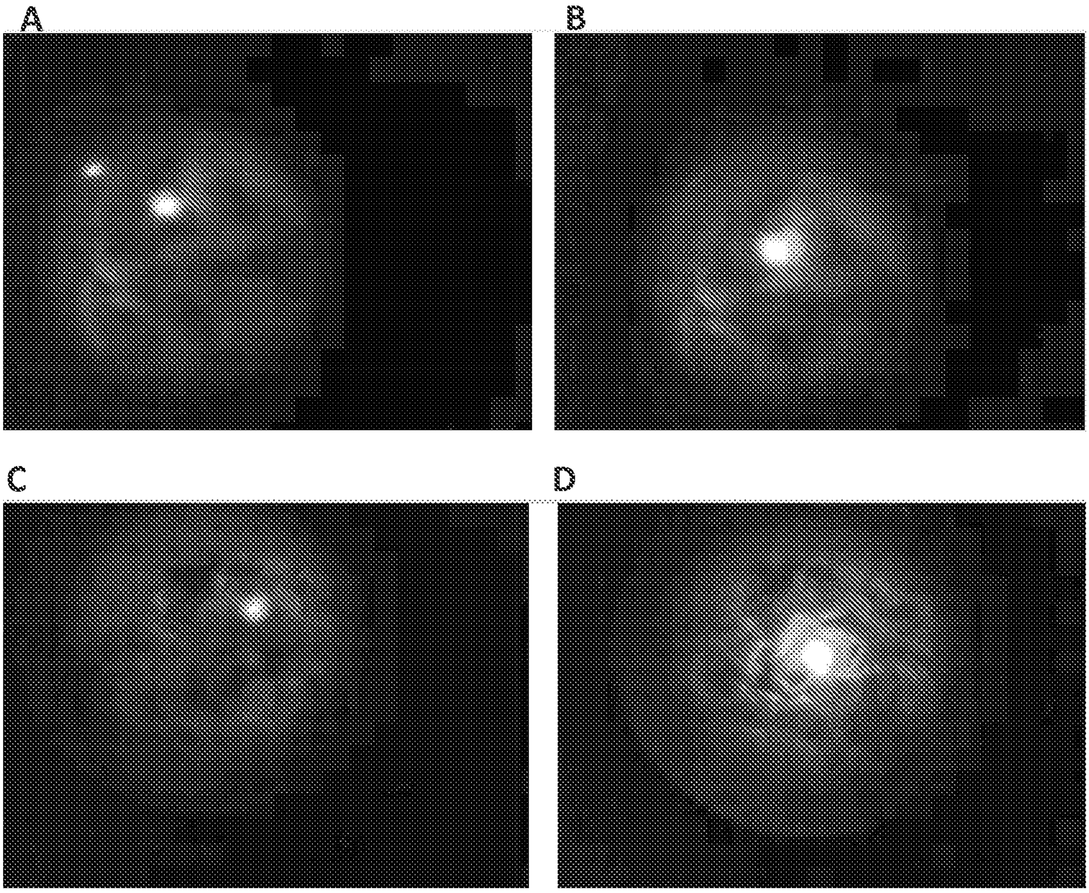


Figures 3A-3D

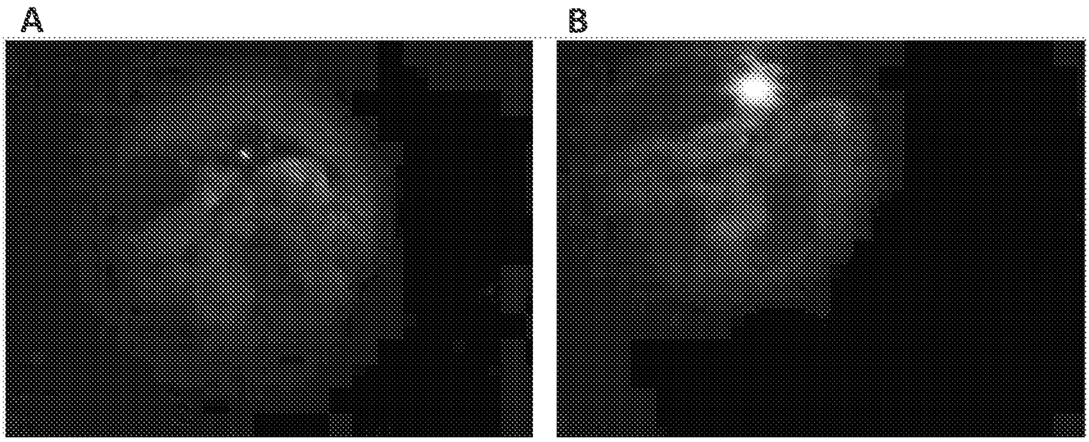


Figures 4A-4B

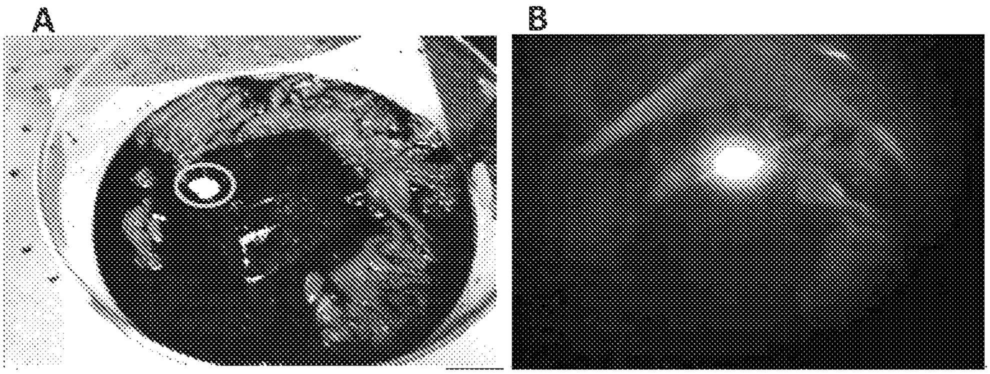


Figures 5A-5B

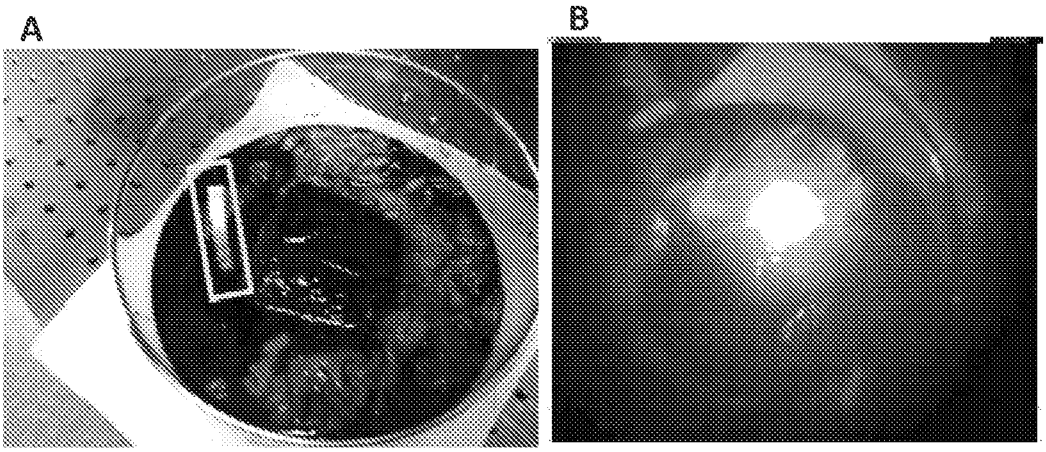


Figures 6A-6B

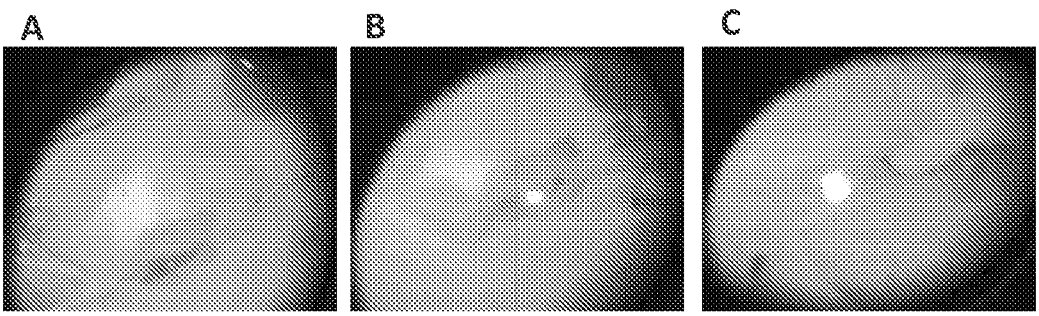


Figures 7A-7C

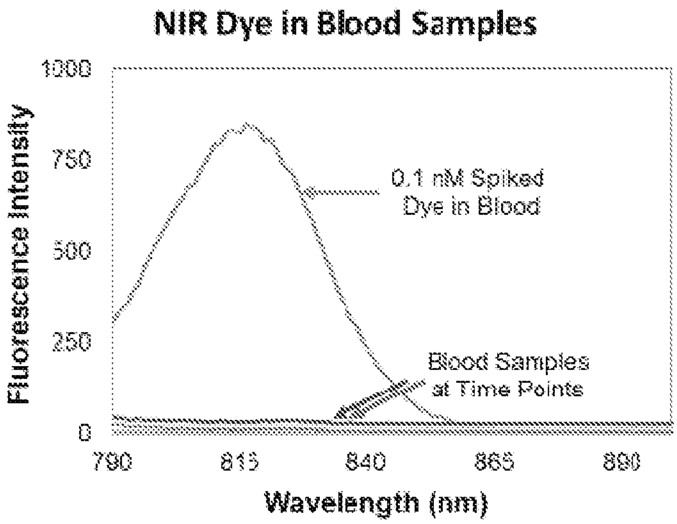


Figures 8

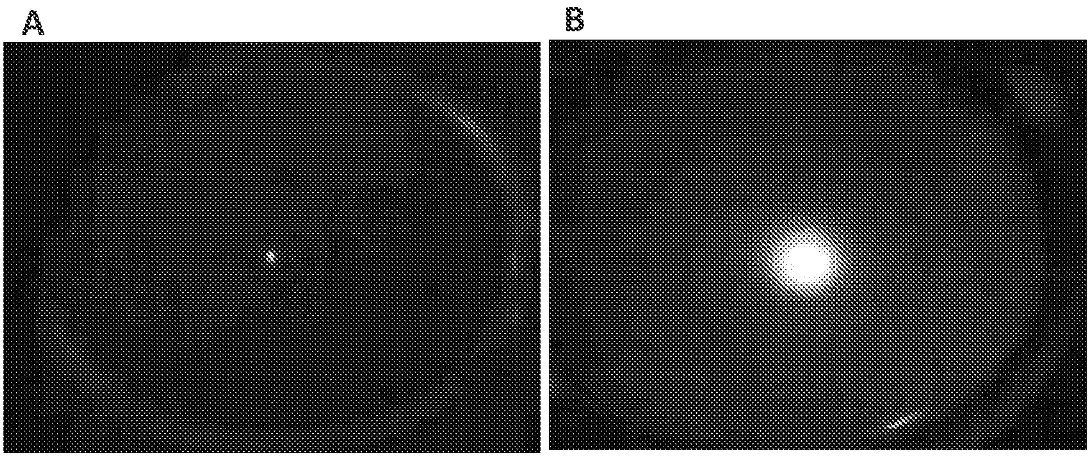


Figures 9A-9B

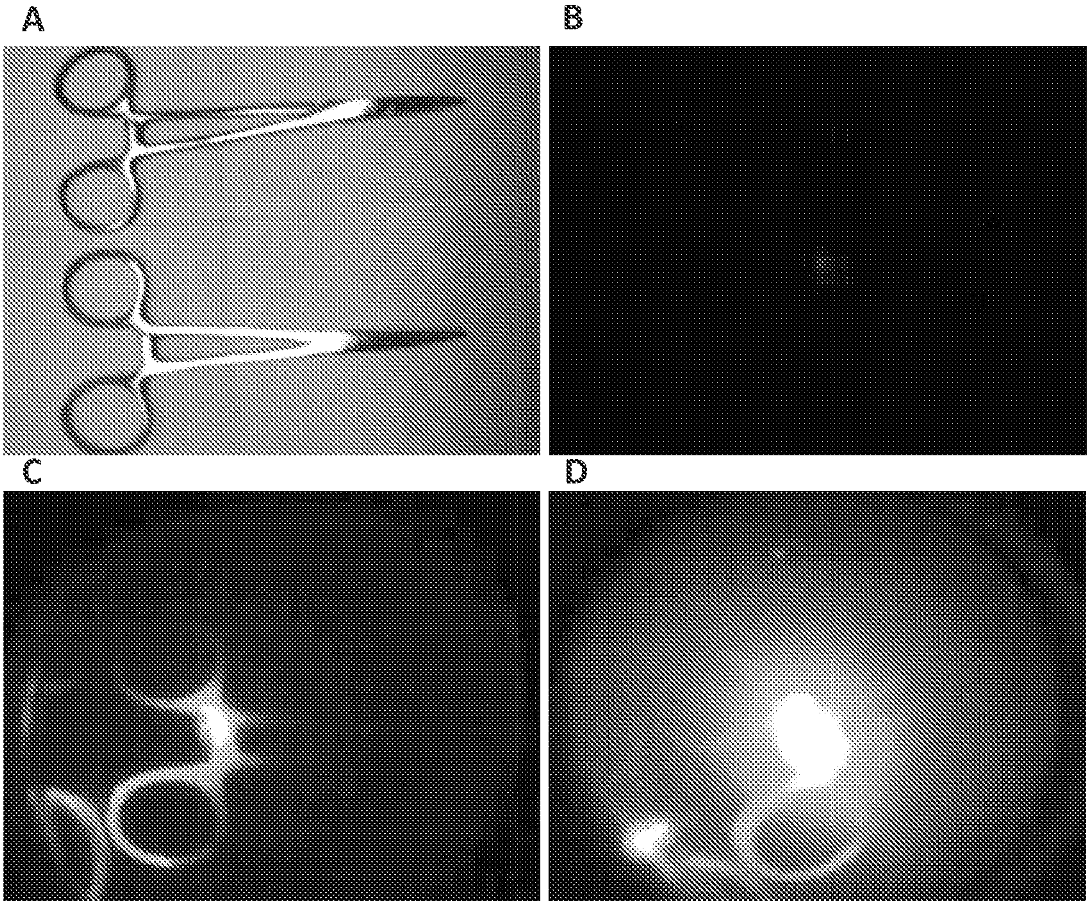


Figures 10A-10D

A

B

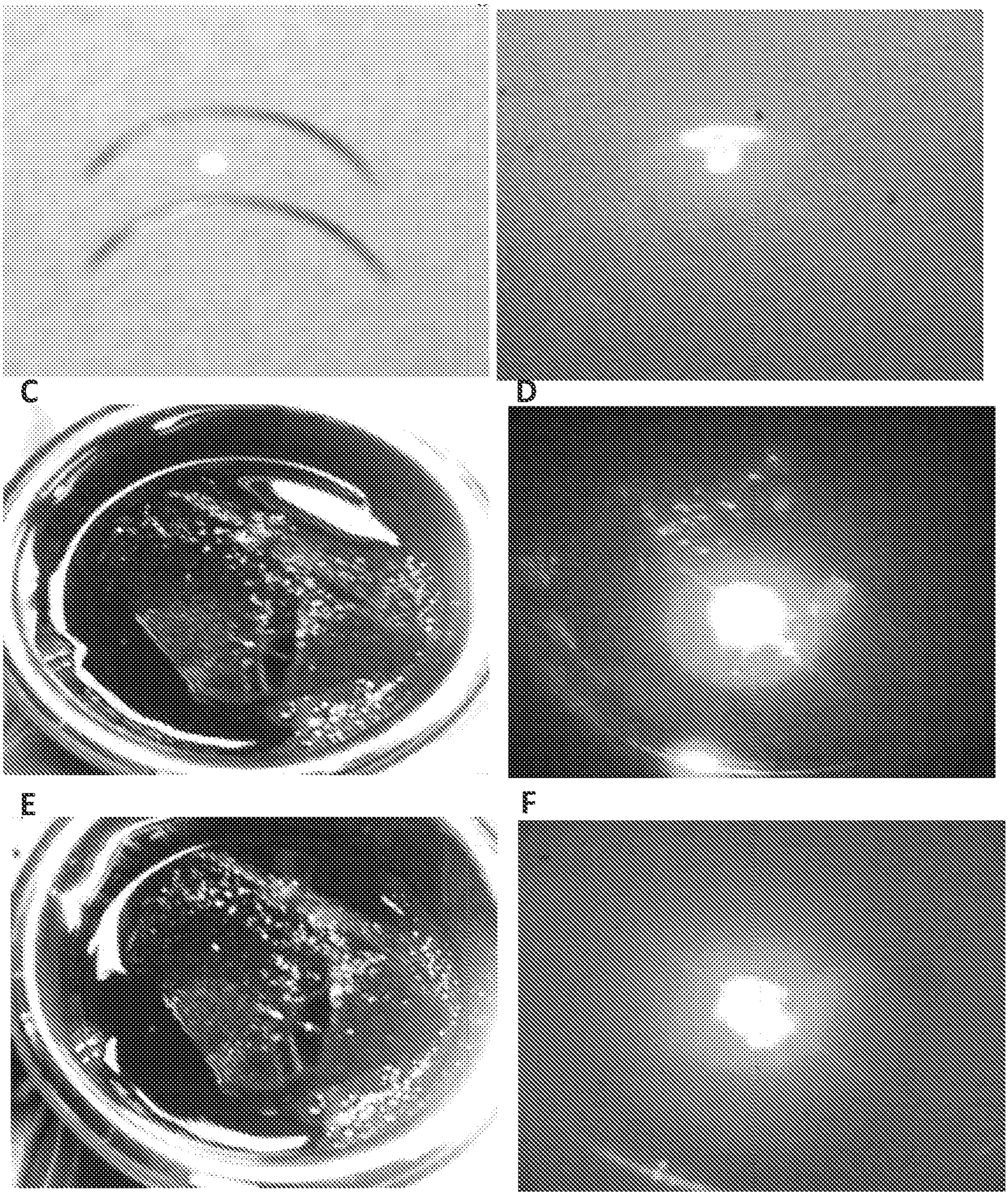


Figures 11A-11F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62078529
- WO 200219918 A
- US 2011163854 A
- US 2014248213 A
- WO 2014144624 A
- US 5625048 A
- US 20090089942 A


### Non-patent literature cited in the description


- **SOWKA M.P.** *Conn Med.,* 1981, vol. 45, 109-115
- **GAWANDE A. et al.** *N Engl J Med.,* 2003, vol. 348, 229-235
- **GREENE et al.** Protective Groups in Organic Synthesis. John Wiley and Sons, 1999
- **HEIM et al.** *Nature,* 1995, vol. 373, 663-664
- **DELAGRAVE et al.** *Biotechnology,* 1995, vol. 13, 151-154
- **CORMACKETAL.** *Gene,* 1996, vol. 173, 33-38
- **CRAMER et al.** *Nature Biotechnol.,* 1996, vol. 14, 315-319